# EUROPEAN PATENT APPLICATION

(11) **EP 4 570 205 A1**
(43) Date of publication of application: **18.06.2025**
(21) Application number: 24215116.5
(22) Date of filing: 25.11.2024
(51) Int. Cl.: A61B 18/12, G16H 20/40, A61B 18/14, A61B 18/00

(54) **ASSISTANCE DEVICE, ASSISTANCE METHOD, AND RECORDING MEDIUM**

(30) Priority: 28.11.2023 US 202363603568 P
(71) Applicant: Olympus Medical Systems Corp., Tokyo 192-8507 (JP)
(72) Inventor: KATO, Gen, Tokyo, 192-8507 (JP); TAKAMI, Sadayoshi, Tokyo, 192-8507 (JP); MATSUDA, Sayaka, Tokyo, 192-8507 (JP); ISHIHATA, Hirotaka, Tokyo, 192-8507 (JP)
(74) Representative: Gunzelmann, Rainer

(57) **Abstract**

An assistance device includes: a processor, the processor being configured to acquire electrical information related to an energy signal for driving an energy device configured to apply energy to a site to perform treatment on the site, input the electrical information to a trained model to cause the trained model to estimate intraoperative information that is related to the site indicated during an operation, determine whether or not accuracy of the intraoperative information is equal to or greater than a fixed value, and perform control according to the intraoperative information when it is determined that the accuracy is equal to or greater than the fixed value.

## Description

### BACKGROUND

### 1. Technical Field

The present disclosure relates to an assistance device, an assistance method, and a recording medium.

### 2. Related Art

In the related art, there is a known energy device that performs treatment by applying treatment energy to a site that is a target for treatment in living tissue (hereinafter, referred to as a "target site") (for example, see International Publication Pamphlet No. WO 2023/286334). In Patent Literature 1, energy and a captured image of the living tissue are input to a trained model that has been trained by using the energy device or a plurality of training purpose tissue images of captured living tissue, image recognition information that is at least one of tissue information related to the living tissue and processing information related to the treatment that is to be performed on the living tissue is allowed to be estimated by the trained model, and an energy output instruction based on the estimated image recognition information is output to a generator that supplies energy to the energy device.

### SUMMARY

Incidentally, various usage scenes are assumed in the energy device. However, in Patent Literature 1 described above, data other than the captured image is not utilized, no study has been conducted in a case where estimation accuracy estimated by the trained model is low, so that there is room for improvement in a method of controlling the energy device.

The present disclosure has been conceived in light of the circumstances described above, and an object thereof is to provide an assistance device, an assistance method, and a recording medium capable of appropriately controlling the energy device.

To solve the problem described above and to achieve the object, an assistance device according to one aspect of the present disclosure includes: a processor, the processor being configured to acquire electrical information related to an energy signal for driving an energy device configured to apply energy to a site to perform treatment on the site, the site being a target for treatment in living tissue, input the electrical information to a trained model to cause the trained model to estimate intraoperative information that is related to the site indicated during an operation, the trained model having been obtained by training a plurality of pieces of training data in each of which at least one of driving information obtained when the energy device is driven, device information indicating a state of the energy device, and site information related to a size, a type, and a state of the site is used as an input parameter and is associated with the intraoperative information that is used as an output parameter, determine whether or not accuracy of the intraoperative information is equal to or greater than a fixed value, and perform control according to the intraoperative information when it is determined that the accuracy is equal to or greater than the fixed value.

An assistance method according to one aspect of the present disclosure is an assistance method implemented by an assistance device that includes a processor, the processor being configured to acquire electrical information related to an energy signal for driving an energy device configured to apply energy to a site to perform treatment on the site, the site being a target for treatment in living tissue, input the electrical information to a trained model to cause the trained model to estimate intraoperative information that is related to the site indicated during an operation, the trained model having been obtained by training a plurality of pieces of training data in each of which at least one of driving information obtained when the energy device is driven, device information indicating a state of the energy device, and site information related to a size, a type, and a state of the site is used as an input parameter and is associated with the intraoperative information that is used as an output parameter, determine whether or not accuracy of the intraoperative information is equal to or greater than a fixed value, and perform control according to the intraoperative information when it is determined that the accuracy is equal to or greater than the fixed value.

A recording medium according to one aspect of the present disclosure is a non-transitory computer-readable recording medium with an executable program stored thereon. The program causes a computer to execute: acquiring electrical information related to an energy signal for driving an energy device configured to apply energy to a site to perform treatment on the site, the site being a target for treatment in living tissue; inputting the electrical information to a trained model to cause the trained model to estimate intraoperative information that is related to the site indicated during an operation, the trained model having been obtained by training a plurality of pieces of training data in each of which at least one of driving information obtained when the energy device is driven, device information indicating a state of the energy device, and site information related to a size, a type, and a state of the site is used as an input parameter and is associated with the intraoperative information that is used as an output parameter; determining whether or not accuracy of the intraoperative information is equal to or greater than a fixed value; and performing control according to the intraoperative information when it is determined that the accuracy is equal to or greater than the fixed value.

The above and other features, advantages and technical and industrial significance of this disclosure will be better understood by reading the following detailed description of presently preferred embodiments of the disclosure, when considered in connection with the accompanying drawings.

### BRIEF DESCRIPTION OF THE DRAWINGS

FIG. 1 is a diagram illustrating a schematic configuration of a medical system according to a first embodiment of the present disclosure;
FIG. 2 is a block diagram illustrating a functional configuration of an assistance device according to the first embodiment of the present disclosure;
FIG. 3 is a flowchart illustrating an outline of a process performed by the assistance device according to the first embodiment of the present disclosure;
FIG. 4 is a block diagram illustrating a functional configuration of an assistance device according to a second embodiment of the present disclosure;
FIG. 5 is a flowchart illustrating an outline of a process performed by the assistance device according to the second embodiment of the present disclosure;
FIG. 6 is a block diagram illustrating a functional configuration of an assistance device according to a third embodiment of the present disclosure;
FIG. 7 is a flowchart illustrating an outline of a process performed by the assistance device according to the third embodiment of the present disclosure;
FIG. 8 is a block diagram illustrating a functional configuration of an assistance device according to a fourth embodiment of the present disclosure;
FIG. 9 is a flowchart illustrating an outline of a process performed by the assistance device according to the fourth embodiment of the present disclosure;
FIG. 10 is a diagram schematically illustrating determination content determined by a determination unit included in the assistance device according to the fourth embodiment of the present disclosure;
FIG. 11 is a diagram illustrating one example of a determination result determined by the determination unit included in the assistance device according to the fourth embodiment of the present disclosure;
FIG. 12 is a block diagram illustrating a functional configuration of an assistance device according to a fifth embodiment of the present disclosure;
FIG. 13 is a flowchart illustrating an outline of a process performed by the assistance device according to the fifth embodiment of the present disclosure;
FIG. 14 is a block diagram illustrating a functional configuration of an assistance device according to a sixth embodiment of the present disclosure;
FIG. 15 is a flowchart illustrating an outline of a process performed by the assistance device according to the sixth embodiment of the present disclosure;
FIG. 16 is a block diagram illustrating a functional configuration of an assistance device according to a seventh embodiment of the present disclosure;
FIG. 17 is a flowchart illustrating an outline of a process performed by the assistance device according to the seventh embodiment of the present disclosure;
FIG. 18 is a block diagram illustrating a functional configuration of an assistance device according to an eighth embodiment of the present disclosure;
FIG. 19 is a flowchart illustrating an outline of a process performed by the assistance device according to the eighth embodiment of the present disclosure;
FIG. 20 is a block diagram illustrating a functional configuration of an assistance device according to a ninth embodiment of the present disclosure;
FIG. 21 is a flowchart illustrating an outline of a process performed by the assistance device according to the ninth embodiment of the present disclosure;
FIG. 22 is a flowchart illustrating an outline of a process performed by the assistance device according to a first modification of the ninth embodiment; and
FIG. 23 is a flowchart illustrating an outline of a process performed by the assistance device according to a second modification of the ninth embodiment;
FIG. 24 is a block diagram illustrating a functional configuration of each of an assistance device according to a treatment tool and a treatment tool control device according to a tenth embodiment.

### DETAILED DESCRIPTION

In the following, a medical system that includes an assistance device according to the present disclosure will be described in detail with reference to the accompanying drawings. However, the invention of the present disclosure is not limited by the embodiments described below. Furthermore, in the drawings used for the following description, shapes, sizes, and positional relationships are only schematically illustrated to an extent that the content of the present disclosure can be understood. Namely, the present disclosure is not limited to only the shapes, the sizes, and the positional relationships illustrated in the drawings. Furthermore, in a description of the drawings, components that are identical to those in drawings are assigned the same reference numerals. In addition, in a description below, a configuration of the medical system according to the present disclosure will be described, and then, a configuration of the endoscope system will be described.

### First Embodiment

### Configuration of medical system

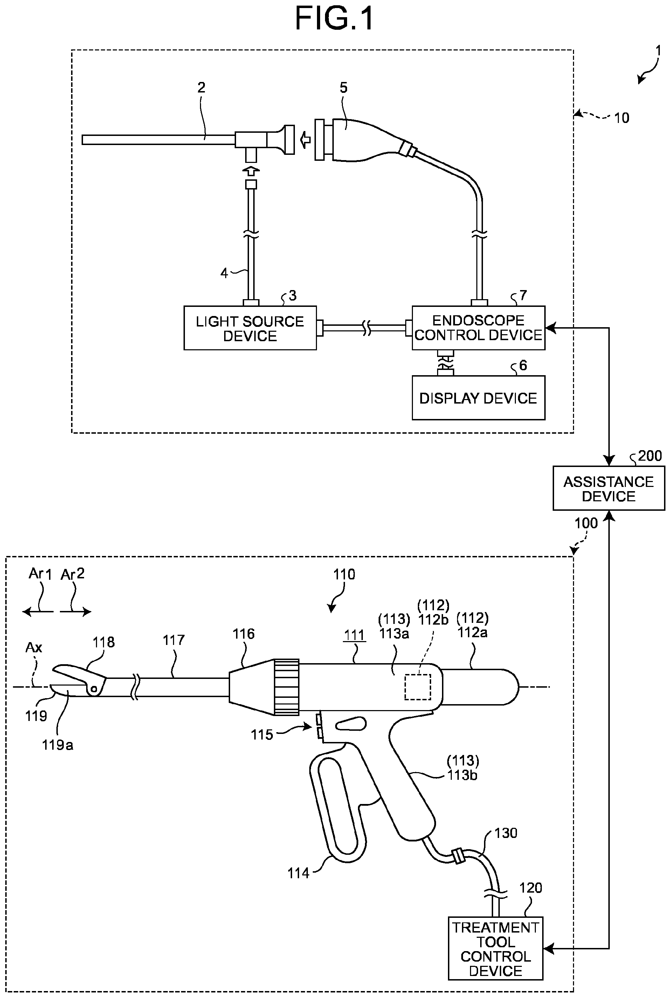

FIG. 1 is a diagram illustrating a schematic configuration of a medical system according to a first embodiment.

A medical system 1 illustrated in FIG. 1 is a system that is used in the medical field, that observes living tissue inside a subject, such as a living body, and that performs treatment on the living tissue by applying energy to a site that is a target for treatment in the living tissue.

As illustrated in FIG. 1, the medical system 1 includes an endoscope system 10 that displays an observation image based on image data that has been obtained by capturing an image of an inside of the subject, a treatment system 100 that performs treatment on living tissue by applying treatment energy to a site that is a target for treatment in the living tissue, and an assistance device 200 that assists the treatment system 100.

### Configuration of endoscope system

First, a configuration of the endoscope system 10 will be described.

The endoscope system 10 includes an insertion portion 2, a light source device 3, a light guide 4, an endoscope camera head 5 (medical imaging device), a display device 6, and an endoscope control device 7.

The insertion portion 2 is rigid or at least a part of the insertion portion 2 is flexible, and the insertion portion 2 has an elongated shape. The insertion portion 2 is inserted into a subject, such as a patient, via a trocar. The insertion portion 2 has, provided therein, an optical system, such as a lens, that forms an observation image.

One end of the light guide 4 is connected to the light source device 3, and, under the control of the endoscope control device 7, the light source device 3 supplies illumination light to be emitted to the interior of a subject to one end of the light guide 4. The light source device 3 is implemented by using at least a light source, a processor having hardware, such as a field programmable gate array (FPGA) or a central processing unit (CPU), and a memory that is a temporary storage area used by the processor. Here, the light source is configured by using one or more of a light emitting diode (LED) light source, a xenon lamp, and a semiconductor laser device, such as laser diode (LD).

The one end of the light guide 4 is detachably connected to the light source device 3, and the other end of the light guide 4 is detachably connected to the insertion portion 2. The light guide 4 guides the illumination light supplied from the light source device 3 to the insertion portion 2.

An eyepiece portion 21 of the insertion portion 2 is detachably connected to the endoscope camera head 5. Under the control of the endoscope control device 7, the endoscope camera head 5 generates an imaging signal (RAW data) by receiving an observation image formed by the insertion portion 2 and performing photoelectric conversion, and outputs the generated imaging signal to the endoscope control device 7. Moreover, in the following description, the configuration including the insertion portion 2 and the endoscope camera head 5 is simply referred to as the endoscope 2.

Under the control of the endoscope control device 7, the display device 6 displays an observation image based on the imaging signal that has been subjected to image processing in the endoscope control device 7, and displays various kinds of information related to the medical system 1. Furthermore, the display device 6 displays various kinds of information that have been input from the assistance device 200 via the endoscope control device 7. The display device 6 is implemented by using a display monitor with, for example, a liquid crystal panel, an organic electroluminescence (EL) panel, or the like.

The endoscope control device 7 is implemented by using a processor having hardware, such as a graphics processing unit (GPU), an FPGA, or a CPU, and a memory that is a temporary storage area used by the processor. The endoscope control device 7 integrally controls the overall operation of the light source device 3, the endoscope camera head 5, and the display device 6 in accordance with a program recorded in the memory. The endoscope control device 7 integrally controls the overall operation of the endoscope system 10 in cooperation with the treatment system 100 and the assistance device 200.

### Outline of treatment system

In the following, a configuration of the treatment system 100 will be described.

The treatment system 100 performs treatment on a site that is a target for treatment in living tissue (hereinafter, simply referred to as a "target site") by applying at least one of ultrasound energy and high frequency energy to the target site. Furthermore, treatment that is practicable by the treatment system 100 according to the first embodiment is treatment for coagulating and sealing the target site, treatment for incising the target site, treatment for performing coagulation and incision at the same time, or the like.

As illustrated in FIG. 1, the treatment system 100 includes a treatment tool 110 and a treatment tool control device 120. Moreover, in FIG. 1, one side along the central axis Ax of the treatment tool 110 will be described as a distal end side Ar1, and the other side will be described as a proximal end side Ar2. Furthermore, in the following description, the treatment system 100 will be explained by using a ultrasound device that is capable of applying ultrasound energy and high frequency energy as one example of the energy device, but the ultrasound device may be a monopolar device that passes high frequency electric power between an electrode provided at the distal end of the device and an electrode provided outside the body or may be a bipolar device that passes high frequency electric power between two jaws.

The treatment tool 110 is an ultrasound treatment tool that performs treatment on the target site by applying ultrasound energy and high frequency energy to the target site, and corresponds to a surgical device. The treatment tool 110 includes a handpiece 111 and an ultrasound transducer 112.

The handpiece 111 includes a holding case 113, a movable handle 114, a switch 115, a rotation knob 116, a pipe 117, a jaw 118, and a vibration transmission member 119.

The ultrasound transducer 112 includes a transducer (TD) case 112a and an ultrasound transducer 112b.

The TD case 112a supports the ultrasound transducer 112b, and is detachably connected to a holding case main body 113a.

The ultrasound transducer 112b generates ultrasonic vibration under the control of the treatment tool control device 120. In the first embodiment, the ultrasound transducer 112b is configured with a bolt-clamped Langevin-type transducer (BLT).

The holding case 113 constitutes an external appearance of the treatment tool 110, and supports the entirety of the treatment tool 110. Furthermore, the holding case 113 includes the holding case main body 113a that is coaxial with the central axis Ax and that has a substantially cylindrical shape, and a fixed handle 113b that extends from the main body of the holding case 113 to a lower part in FIG. 1 and that is held by an operator, such as an operating surgeon.

The movable handle 114 receives opening/closing operation performed by the operator, such as the operating surgeon. The opening/closing operation is an operation of causing the jaw 118 to open and close with respect to an end portion 119a of the vibration transmission member 119 on the distal end side Ar1.

The switch 115 is arranged so as to be exposed to the outside from the side surface of the fixed handle 113b positioned on the distal end side Ar1. Further, the switch 115 receives a treatment operation performed by an operator, such as an operating surgeon. The treatment operation is an operation of applying ultrasound energy or high frequency energy to the target site. In a case where the switch 115 includes a plurality of buttons, an operation instruction is allocated to each of the buttons.

The rotation knob 116 has a substantially cylindrical shape that is coaxial with the central axis Ax, and is arranged on the distal end side Ar1 of the holding case main body 113a. Further, the rotation knob 116 receives a rotation operation performed by an operator, such as an operating surgeon. With the rotation operation, the rotation knob 116 rotates about the central axis Ax relative to the holding case main body 113a. Furthermore, with the rotation of the rotation knob 116, the pipe 117, the jaw 118, and the vibration transmission member 119 rotate about the central axis Ax.

The pipe 117 is a cylindrical pipe. A pin (not illustrated) that pivotally supports the jaw 118 such that the jaw 118 is rotatable about the central axis Ax is fixed at the end portion of the pipe 117 positioned on the distal end side Ar1.

At least a part of the jaw 118 is constituted of an electrically conductive material. Further, the jaw 118 opens and closes relative to the end portion 119a of the vibration transmission member 119 positioned on the distal end side Ar1 in accordance with a grasping operation that is performed on the movable handle 114 by an operator, such as an operating surgeon, and grasps a target site with the end portion 119a.

The vibration transmission member 119 is constituted of an electrically conductive material, and has an elongated shape linearly extending along the central axis Ax. Moreover, the vibration transmission member 119 is inserted in the pipe 117 in a state in which the end portion 119a positioned on the distal end side Ar1 protrudes outside the pipe 117. At this time, although not specifically illustrated in the drawing, the end portion of the vibration transmission member 119 positioned on the proximal end side Ar2 is mechanically connected to the ultrasound transducer 112. That is, the vibration transmission member 119 transmits ultrasound vibrations generated by the ultrasound transducer 112 from the end portion positioned on the proximal end side Ar2 to the end portion 119a positioned on the distal end side Ar1. In the first embodiment, the ultrasound vibrations are longitudinal vibrations that vibrate in a direction along the central axis Ax.

Under the control of the assistance device 200, the treatment tool control device 120 integrally controls an operation of the treatment tool 110 by way of an electrical cable 130. Specifically, the treatment tool control device 120 detects a treatment operation performed on the switch 115 by an operator, such as an operating surgeon. Further, in a case where the treatment tool control device 120 has detected the treatment operation performed by the operator, the treatment tool control device 120 applies, by way of the electrical cable 130, ultrasound energy or high frequency energy to the target site that is grasped between the jaw 118 and the end portion 119a of the vibration transmission member 119 that is positioned on the distal end side Ar1. In other words, the treatment tool control device 120 performs treatment on the target site by way of the treatment tool 110.

For example, in a case where the treatment tool control device 120 applies ultrasound energy to the target site, the treatment tool control device 120 supplies, under the control of the assistance device 200, driving electric power to the ultrasound transducer 112b by way of the electrical cable 130. Accordingly, the ultrasound transducer 112b generates longitudinal vibrations (ultrasound vibrations) vibrating in the direction along the central axis Ax. Furthermore, the end portion 119a of the vibration transmission member 119 positioned on the distal end side Ar1 vibrates at a desired amplitude caused by the longitudinal vibrations. Further, ultrasound vibrations are applied from the end portion 119a to the target site that is grasped between the jaw 118 and the end portion 119a. In other words, ultrasound energy is applied from the end portion 119a to the target site.

Furthermore, for example, in a case where the treatment tool control device 120 applies high frequency energy to the target site, the treatment tool control device 120 supplies, under the control of the assistance device 200, high frequency electric power between the jaw 118 and the vibration transmission member 119 by way of the electrical cable 130. Accordingly, a high frequency electric current flows through the target site that is grasped between the jaw 118 and the end portion 119a of the vibration transmission member 119 positioned on the distal end side Ar1. In other words, high frequency energy is applied to the target site.

Furthermore, the treatment tool control device 120 is connected to the endoscope control device 7 and the assistance device 200 so as to be able to communicate each other, and outputs a signal indicating a press of the switch to the endoscope control device 7, when the switch 115 has been pressed. In addition, the treatment tool control device 120 outputs electrical information related to an energy signal for driving the treatment tool 110 to the assistance device 200, and receives a control parameter or the like that is used to drive the treatment tool 110 and that is input from the assistance device 200. Here, the electrical information indicates time series data on at least one of a US signal and a High Frequency (HF) signal for driving the treatment tool 110. Of course, in the electrical information, a voltage, an electric current, electric power, impedance, a phase, or the like may be included. Furthermore, in the control parameter, a voltage, an electric current, electric power to be supplied, a phase, output time, and the like are included.

Configuration of assistance device

In the following, a functional configuration of the assistance device 200 will be described.

The assistance device 200 outputs various kinds of information for assisting the endoscope system 10 and the treatment system 100 to the endoscope system 10 and the treatment system 100. Specifically, the assistance device 200 acquires various kinds of information from the endoscope system 10 and the treatment system 100, estimates, on the basis of the various kinds of acquired information, a control parameter for assisting the endoscope system 10 and the treatment system 100, and outputs the estimated control parameter to the endoscope system 10 and the treatment system 100.

### Functional configuration of assistance device

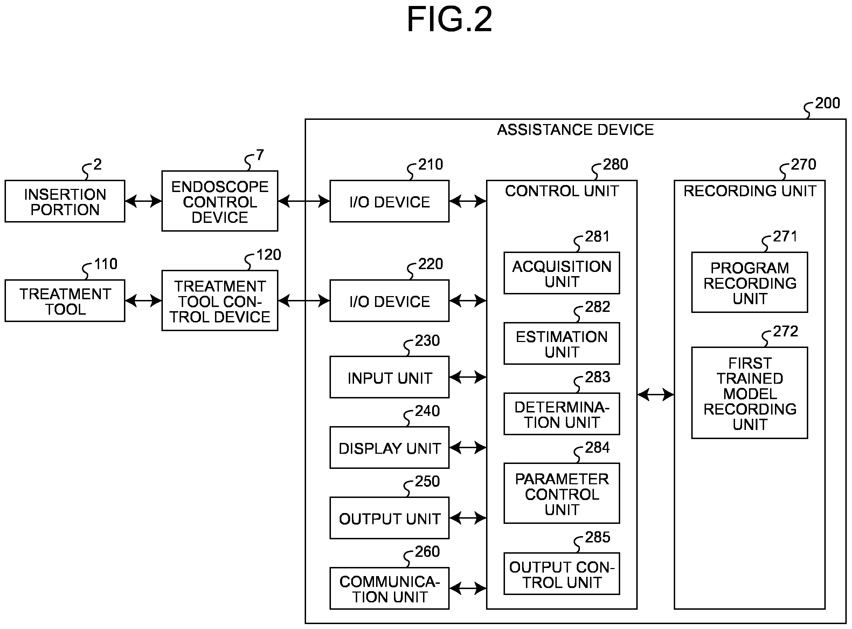

In the following, a functional configuration of the assistance device 200 will be described. FIG. 2 is a block diagram illustrating the functional configuration of the assistance device 200.

The assistance device 200 illustrated in FIG. 2 includes an input/output (I/O) device 210, an I/O device 220, an input unit 230, a display unit 240, an output unit 250, a communication unit 260, a recording unit 270, and a control unit 280.

The I/O device 210 receives image data and the various kinds of information that are output from the endoscope control device 7, and outputs the received image data and the various kinds of information to the control unit 280. Furthermore, the I/O device 210 outputs the various kinds of information that have been input from the control unit 280 to the endoscope control device 7. The I/O device 210 is configured by use of an interface circuit or the like including a connector to which, for example, a cable capable of image transmission and communication is connected.

The I/O device 220 receives the electrical information and the various kinds of information that are output from the treatment tool control device 120, and outputs the received electrical information and the various kinds of information to the control unit 280. Furthermore, the I/O device 220 outputs, to the treatment tool control device 120, various kinds of information including the control parameter that is used to perform treatment on the target site by the treatment tool 110 and that has been input from the control unit 280. The I/O device 220 is configured by use of an interface circuit or the like including a connector to which, for example, a cable capable of image transmission and communication is connected.

The input unit 230 receives an input operation performed by an operator, such as an operating surgeon, and outputs various signals according to the received input operation to the control unit 280. The input unit 230 is configured by use of an input device, such as a touch panel, a switch, a keyboard, a mouse, or a button.

The display unit 240 displays, under the control of the control unit 280, various kinds of information related to the assistance device 200. The display unit 240 is configured by use of a display monitor with, for example, an organic EL panel, a liquid crystal panel, or the like.

The output unit 250 outputs, under the control of the control unit 280, various kinds of information related to the assistance device 200. The output unit 250 is configured by use of, for example, a speaker, or the like.

The communication unit 260 transmits, under the control of the control unit 280, the various kinds of information that are input from the control unit 280 to an external server, or the like, and outputs the various kinds of information that have been received from the external server to the control unit 280. The communication unit 260 is configured by use of a communication module or the like capable of performing wireless communication using, for example, Wi-Fi (registered trademark) or Bluetooth (registered trademark).

The recording unit 270 records various kinds of information related to the assistance device 200. The recording unit 270 is configured by use of a hard disk drive (HDD), a solid state drive (SSD), a flash memory, a volatile memory, a nonvolatile memory, or the like. The recording unit 270 includes a program recording unit 271 and a first trained model recording unit 272.

The program recording unit 271 records various kinds of programs executed by the assistance device 200 and data that is being processed.

The first trained model recording unit 272 records a first trained model. Specifically, the first trained model has been trained by using an already known training model and using a plurality of pieces of training data in each of which at least one of driving information obtained when the energy device including the treatment tool 110 and the like is driven, device information that indicates a state of the energy device, and site information related to a type of a target site, a size of the target site, and a state of the target site is used as input parameter and is associated with intraoperative information that is related to the target site indicated during an operation and that is used as an output parameter.

The driving information includes a voltage of the energy device, an electric current, electric power to be supplied, impedance, driving time, a phase, or the like indicated when the energy device is driven.

The device information includes a driving state, stopping information, a treatment state, a grasping state of the target site grasped by the energy device, or the like. Examples of the grasping state of the target site include a normal grasp, a diagonal grasp, and a shift in grasp position relative to the target site. For example, the grasping state of the target site is a value based on the impedance of the target site grasped between the jaw 118 and the end portion 119a, a contact area of the target site positioned at the end portion 119a, or the like.

The type of the target site includes the type of a blood vessel or living tissue, the type of mucosa, the type of a tumor, the degree of progress of the tumor, or the like.

The size of the target site includes a size of a blood vessel, a thickness of the blood vessel, a diameter of the blood vessel, a pressure resistance value of the blood vessel, or the like.

The state of the target site includes a type of treatment that is being performed by the energy device (for example, a sealed state, bleeding information, or hemostasis information) and a state of the living tissue in the circumference environment. The circumference environment of the living tissue includes an amount of connective tissue in the vicinity of the target site, an amount of moisture, or the like.

The intraoperative information includes a control parameter for controlling the energy device when the energy device (the treatment tool 110) applies energy to the target site, treatment information on treatment performed by the energy device, state information that indicates a state of the target site subjected to treatment performed by the energy device, the accuracy of an estimation result (output parameter), or the like.

The control parameter includes a parameter of each of a voltage, an electric current, high frequency energy, ultrasound energy, and the like to be supplied to the treatment tool 110 by the treatment tool control device 120 in order to perform optimum treatment on the target site by the treatment tool 110.

The treatment information includes the content, such as a grasp, incision, and sealing, of the treatment performed on the target site by the energy device (the treatment tool 110).

The state information includes site information indicating whether or not the target site has been incised by the energy device (the treatment tool 110) and site information that is related to a bleeding state, risk of bleeding, the diameter of a blood vessel, a pressure resistance value of the blood vessel, and the target site.

The accuracy is the accuracy of an incision state of the target site output by the first trained model including, for example, the accuracy rate indicated by %, an evaluation value, an AI score, or the like.

Furthermore, a trained model that is generated by machine learning is used for the first trained model. Specifically, an example of the first trained model used includes a trained model that is generated from various kinds of machine learning techniques of deep learning using neural networks, support vector machines, a decision tree, naive Bayes classifiers, the k-nearest neighbors algorithm, or the like. Moreover, in a case where neural networks are used for the first trained model, a convolutional neural network (CNN) may be used. That is, the first trained model includes an input layer to which input parameters including the driving information obtained when the energy device including the treatment tool 110 or the like is driven, the device information indicating a state of the energy device, and the site information related to the type of the target site, the size of the target site, and the state of the target site are input; a plurality of intermediate layers in each of which arithmetic processing is performed on the data that has been input through the input layer; and an output layer that outputs, as output parameters, the intraoperative information related to the living tissue recognized during an operation on the basis of the arithmetic processing results that have been output from these plurality of intermediate layers.

The control unit 280 is implemented by a processor including hardware, such as a GPU, a digital signal processor (DSP), an FPGA, or a CPU, and a memory that is a temporary storage area used by the processor. The control unit 280 controls each of the units constituting the assistance device 200. The control unit 280 includes an acquisition unit 281, an estimation unit 282, a determination unit 283, a parameter control unit 284, and an output control unit 285.

The acquisition unit 281 acquires electrical information from the treatment tool control device 120 included in the treatment system 100 via the I/O device 220. Specifically, the acquisition unit 281 acquires, from the treatment tool control device 120, electrical information that is provided, in time series, when the treatment tool control device 120 applies at least one of the high frequency energy signal and the ultrasound energy signal to be supplied to the treatment tool 110 to the target site.

The estimation unit 282 estimates the intraoperative information indicated during an operation on the basis of both of the first trained model and the electrical information that has been acquired by the acquisition unit 281. Specifically, the estimation unit 282 inputs the electrical information acquired by the acquisition unit 281 to the first trained model as an input parameter, and estimates an output parameter that is output from the first trained model as the intraoperative information.

The determination unit 283 determines whether or not the accuracy included in the intraoperative information estimated by the estimation unit 282 is equal to or greater than a fixed value.

The parameter control unit 284 changes the control parameter for controlling the treatment tool 110 performed by the treatment tool control device 120 to a control parameter that is included in the intraoperative information estimated by the estimation unit 282, and outputs the changed control parameter.

The output control unit 285 outputs information indicating that the control parameter has been changed to the display unit 240 or the output unit 250.

### Process performed by assistance device

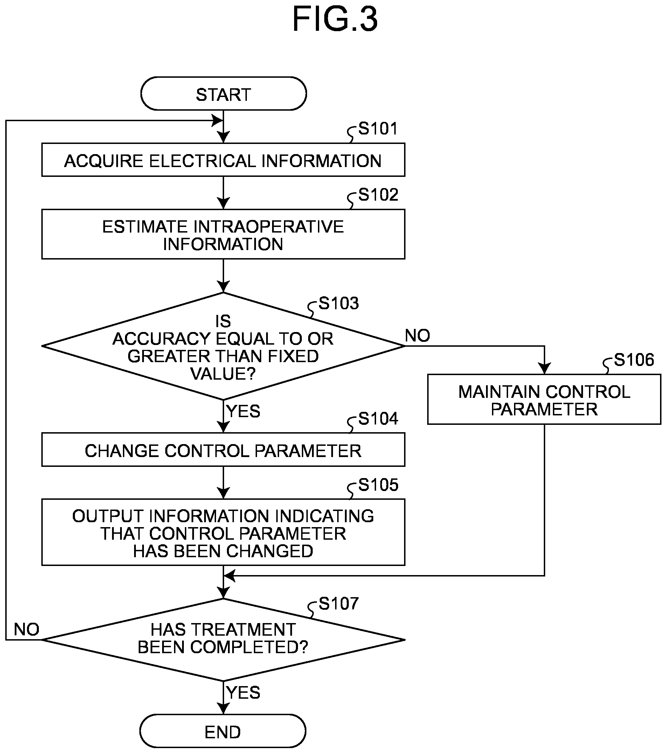

In the following, the process performed by the assistance device 200 will be described. FIG. 3 is a flowchart illustrating an outline of the process performed by the assistance device 200.

As illustrated in FIG. 3, first, the acquisition unit 281 acquires the electrical information from the treatment tool control device 120 included in the treatment system 100 via the I/O device 220 (Step S101). Specifically, the acquisition unit 281 acquires, from the treatment tool control device 120, the electrical information that is provided, in time series, when the treatment tool control device 120 applies at least one of the high frequency energy signal and the ultrasound energy signal to be supplied to the treatment tool 110 to the target site. More specifically, the electrical information is time series data on at least one of the US signal and the HF signal that is supplied to the treatment tool 110 by the treatment tool control device 120. Of course, the electrical information may include a voltage, an electric current, and driving electric power that are supplied to the treatment tool 110 by the treatment tool control device 120, and include the driving time, the impedance information, the phase information, and the like.

Subsequently, the estimation unit 282 estimates, on the basis of both of the first trained model and the electrical information that has been acquired by the acquisition unit 281, the intraoperative information the is related to the target site indicated during an operation (Step S102). Specifically, the estimation unit 282 inputs the electrical information acquired by the acquisition unit 281 to the first trained model as the input parameter, and estimates the output parameter that is output from the first trained model as the intraoperative information related to the target site indicated during the operation.

After that, the determination unit 283 determines whether or not the accuracy included in the intraoperative information estimated by the estimation unit 282 is equal to or greater than the fixed value (Step S103). Here, the fixed value is, for example, 80% or above. In this case, the determination unit 283 determines whether or not the accuracy included in the intraoperative information estimated by the estimation unit 282 is equal to or greater than 80%. Moreover, the fixed value may be appropriately set by the user, but, in order to maintain the estimation accuracy, the fixed value is preferably set to 80% or above, and is more preferably set to 90% or above. If it is determined by the determination unit 283 that the accuracy included in the intraoperative information estimated by the estimation unit 282 is equal to or greater than the fixed value (Yes at Step S103), the assistance device 200 proceeds to Step S104 that will be described later. In contrast, if it is determined by the determination unit 283 that the accuracy included in the intraoperative information estimated by the estimation unit 282 is not equal to or greater than the fixed value (No at Step S103), the assistance device 20 proceeds to Step S105 that will be described later.

At Step S104, the parameter control unit 284 changes, on the basis of the intraoperative information estimated by the estimation unit 282, the control parameter for controlling the treatment tool 110 performed by the treatment tool control device 120, and outputs the changed control parameter to the treatment tool control device 120. Specifically, the parameter control unit 284 changes the control parameter for controlling the treatment tool 110 by the treatment tool control device 120 to the control parameter that is included in the intraoperative information estimated by the estimation unit 282. For example, the parameter control unit 284 outputs, to the treatment tool control device 120, the control parameter that is used to increase or decrease an output electric current to be supplied to the treatment tool 110 by the treatment tool control device 120. Of course, the parameter control unit 284 may stop the output electric current that is supplied by the treatment tool control device 120 to the treatment tool 110. As a result of this, the treatment tool control device 120 is able to supply, to the treatment tool 110, the high frequency energy signal and the ultrasound energy signal for performing optimum treatment on the target site that is being subjected to treatment performed by the treatment tool 110. Consequently, the operator is able to perform optimum treatment on the target site by using the treatment tool 110.

Subsequently, the output control unit 285 outputs information indicating that the control parameter has been changed to the display unit 240 or the output unit 250 (Step S105). In this case, the output control unit 285 may cause the display unit 240 to display, on the basis of the state information included in the intraoperative information estimated by the estimation unit 282, a risk of bleeding on the target site (for example, establishment of the risk of bleeding), the diameter of the blood vessel, the pressure resistance value of the blood vessel, the site, and the like, or may cause the output unit 250 to output a warning beep. As a result of this, the operator is able to recognize that the control parameter has been changed. After the process performed at Step S105, the assistance device 200 proceeds to Step S107 that will be described later. Furthermore, the assistance device 200 may proceed to Step S106 by omitting the process performed at Step S105 in accordance with the display setting content at the time of the change in the control parameter that has been set to the assistance device 200 by the operator.

At Step S106, the parameter control unit 284 maintains the control parameter for controlling the treatment tool 110 performed by the treatment tool control device 120. Specifically, the parameter control unit 284 maintains the control parameter for controlling the treatment tool 110 performed by the treatment tool control device 120 without using the control parameter that is included in the intraoperative information estimated by the estimation unit 282. As a result of this, it is possible to prevent the control parameter from being automatically changed in a low reliable state of the estimation result when the accuracy of the estimation result estimated by the estimation unit 282 is less than the fixed value. After the process performed at Step S106, the assistance device 200 proceeds to Step S107 that will be described later.

At Step S107, the determination unit 283 determines whether or not the operator has completed the treatment performed on the subject. If it is determined, by the determination unit 283, that the operator completes the treatment performed on the subject (Yes at Step S107), the assistance device 200 completes the process. In contrast, if it is determined, by the determination unit 283, that the operator has not completed the treatment performed on the subject (No at Step S107), the assistance device 200 returns to Step S101 described above.

According to the first embodiment as described above, if it is determined, by the determination unit 283, that the accuracy that is included in the intraoperative information estimated by the estimation unit 282 is equal to or greater than the fixed value, the parameter control unit 284 changes, on the basis of the intraoperative information estimated by the estimation unit 282, the control parameter for controlling the treatment tool 110 performed by the treatment tool control device 120, and outputs the changed control parameter to the treatment tool control device 120. As a result of this, the operator is able to appropriately control the treatment tool 110.

Furthermore, according to the first embodiment, if it is determined, by the determination unit 283, that the accuracy that is included in the intraoperative information estimated by the estimation unit 282 is equal to or greater than the fixed value, the parameter control unit 284 maintains the control parameter for controlling the treatment tool 110 performed by the treatment tool control device 120, so that it is possible to prevent the control parameter from being automatically changed in a low reliable state of the estimation result when the accuracy of the estimation result estimated by the estimation unit 282 is less than the fixed value.

Furthermore, according to the first embodiment, the output control unit 285 outputs information indicating that the control parameter has been changed to the display unit 240 or the output unit 250, so that the operator is able to intuitively recognize that the control parameter that is used when the treatment tool 110 applies energy to the target site.

### Second Embodiment

In the following, a second embodiment will be described. In the second embodiment, determination is performed on the basis of the accuracy of each of the plurality of estimation results. Accordingly, in the following description, after the functional configuration of an assistance device according to the second embodiment has been described, the process performed by the assistance device according to the second embodiment will be described. Furthermore, components that are the same as those included in the assistance device 200 according to the above described first embodiment are assigned the same reference numerals and descriptions thereof in detail will be omitted.

### Functional configuration of assistance device

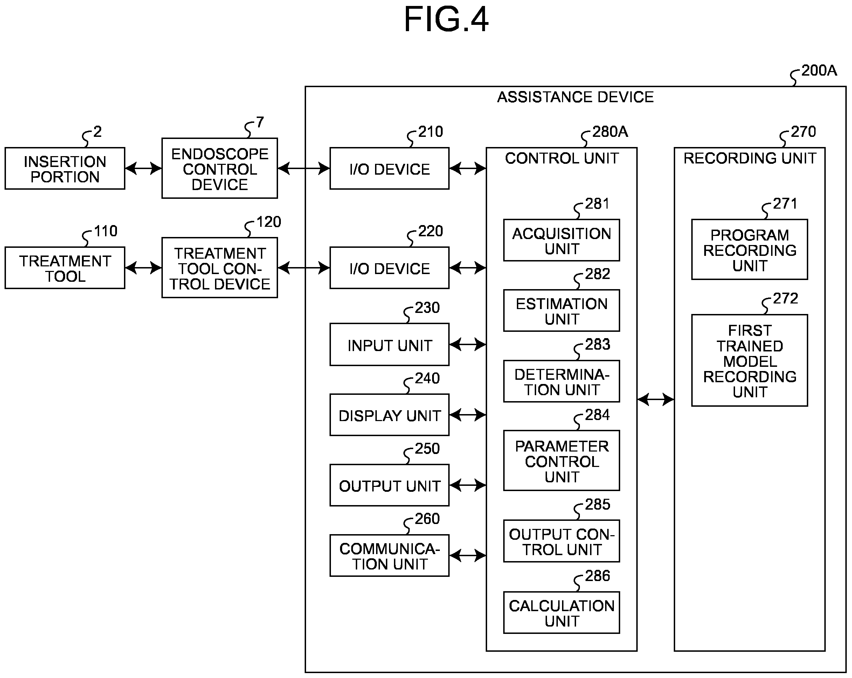

FIG. 4 is a block diagram illustrating a functional configuration of the assistance device according to the second embodiment. An assistance device 200A illustrated in FIG. 4 includes a control unit 280A instead of the control unit 280 included in the assistance device 200 according to the above described first embodiment. The control unit 280A further includes a calculation unit 286, in addition to the functional configuration of the control unit 280 according to the first embodiment.

The calculation unit 286 calculates the accuracy of the intraoperative information on the basis of the intraoperative information indicated by each of the plurality of estimation results obtained by the estimation unit 282 estimated a predetermined number of times. For example, the calculation unit 286 calculates an average value of the accuracy included in the intraoperative information indicated by each of the estimation results obtained by the estimation unit 282 estimated ten times as the accuracy that is to be compared with the fixed value.

### Process performed by assistance device

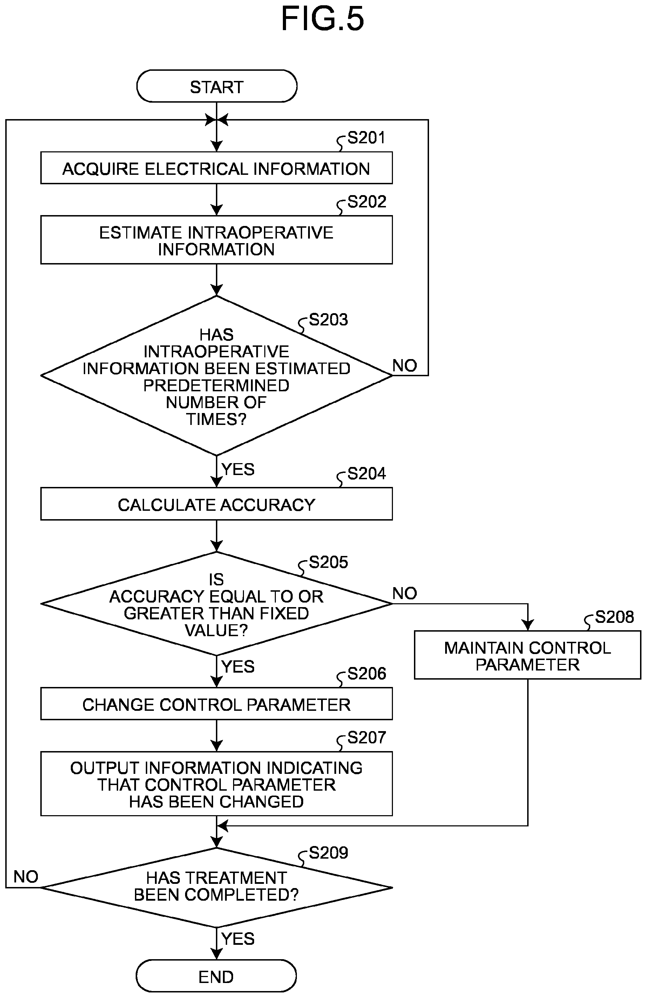

In the following, a process performed by the assistance device 200A will be described. FIG. 5 is a flowchart illustrating an outline of the process performed by the assistance device 200A. In FIG. 5, Step S201, Step S202, and Step S206 to Step S209 correspond to Step S101, Step S102, and Step S104 to Step S107, respectively, illustrated in FIG. 3 described above, and the same processes are performed at these steps; therefore, descriptions thereof in detail will be omitted. Accordingly, in the following description, each of the processes performed by the assistance device 200A at Step S203 to Step S205 will be described.

At Step S203, the determination unit 283 determines whether or not the estimation unit 282 has estimated the intraoperative information a predetermined number of times. Specifically, the determination unit 283 determines whether or not the estimation unit 282 has estimated the intraoperative information, for example, 10 or more times. If it is determined, by the determination unit 283, that the estimation unit 282 has estimated the intraoperative information a predetermined number of times (Yes at Step S203), the assistance device 200A proceeds to Step S204 that will be described later. In contrast, if it is determined, by the determination unit 283, that the estimation unit 282 has not estimated the intraoperative information the predetermined number of times (No at Step S203), the assistance device 200A returns to Step S201.

At Step S204, the calculation unit 286 calculates the accuracy of the intraoperative information on the basis of the intraoperative information indicated by each of the plurality of estimation results obtained by the estimation unit 282 estimated the predetermined number of times. Specifically, the calculation unit 286 calculates, as the accuracy, the average value of the accuracy that is included in the intraoperative information indicated by each of the estimation results obtained by the estimation unit 282 estimated 10 times. For example, in a case where the accuracy is output by using a probability denoted by % that indicates the completion of incision of the target site included in the intraoperative information indicated by each of the estimation results obtained by the estimation unit 282 estimated 10 times, the calculation unit 286 calculates, as the accuracy, the average value obtained by adding each of the probabilities and dividing the obtained probability by the number of times. Of course, the calculation unit 286 may calculate not only the average value, but also a median value or a mode value as the accuracy, or may calculate a standard deviation, the maximum value, the minimum value, the number of times of outliers, or the like as the accuracy. Furthermore, in addition to the case where the accuracy is output by the probability denoted by % that indicates the completion of incision of the target site included in the intraoperative information indicated by each of the estimation results obtained by the estimation unit 282 estimated 10 times, in a case of a pressure resistance value or the size of a blood vessel, the calculation unit 286 may calculate the median value or the mode value as the accuracy.

The determination unit 283 determines whether or not the accuracy calculated by the calculation unit 286 is equal to or greater than the fixed value (Step S205). If it is determined, by the determination unit 283, that the accuracy calculated by the calculation unit 286 is equal to or greater than the fixed value (Yes at Step S205), the assistance device 200A proceeds to Step S206. In contrast, if it is determined, by the determination unit 283, that the accuracy calculated by the calculation unit 286 is not equal to or greater than the fixed value (No at Step S205), the assistance device 200A proceeds to Step S207.

According to the above described second embodiment, if it is determined, by the determination unit 283, that the accuracy calculated by the calculation unit 286 is equal to or greater than the fixed value, the parameter control unit 284 changes, on the basis of the intraoperative information that has been estimated by the estimation unit 282, the control parameter for controlling the treatment tool 110 performed by the treatment tool control device 120, and outputs the changed control parameter to the treatment tool control device 120. As a result of this, the operator is able to appropriately control the treatment tool 110 by using the control parameter having a high reliable estimation result of the first trained model.

### Third Embodiment

In the following, a third embodiment will be described. In the third embodiment, the accuracy is determined by being calculated on the basis of a plurality of pieces of intraoperative information estimated by using each of a plurality of trained models that have been obtained by training the training data in different situations. Accordingly, in the following description, after a functional configuration of an assistance device according to the third embodiment has been described, the process performed by the assistance device according to the third embodiment will be described. Furthermore, components that are the same as those included in the assistance devices 200 and 200A according to the first and the second embodiments, respectively, described above are assigned the same reference numerals and descriptions thereof in detail will be omitted.

### Functional configuration of assistance device

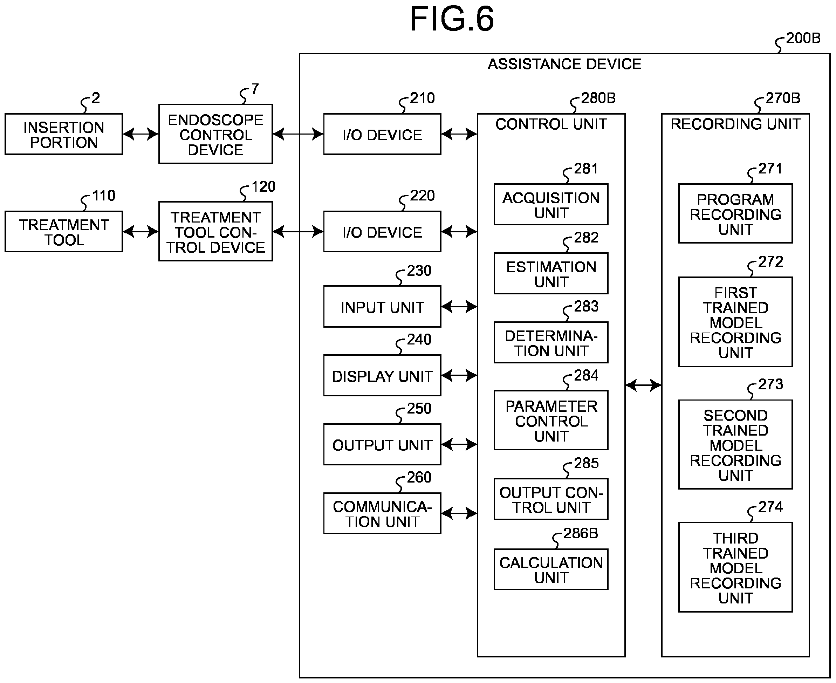

FIG. 6 is a block diagram illustrating the functional configuration of the assistance device according to the third embodiment. An assistance device 200B illustrated in FIG. 6 includes a recording unit 270B and a control unit 280B, instead of the recording unit 270 and the control unit 280A that are included in the assistance device 200A according to the above described second embodiment.

The recording unit 270B further includes a second trained model recording unit 273 and a third trained model recording unit 274, in addition to the functional configuration of the recording unit 270 according to the above described first embodiment.

A second trained model recording unit 273 records a second trained model. Specifically, the second trained model is a trained model that has been trained by using an already known training model and using a plurality of pieces of training data each of which is obtained in a different situation from that used for the first trained model and in each of which the driving information, the device information, and the site information that are used as input parameters and are associated with the intraoperative information that is used as an output parameter. In the second trained model, the trained model that has been generated by using the same machine learning technique as that used for the first trained model is used, and the same training model is used, except that training data to be trained and a weighting factor or the like in the intermediate layer are different from those used in the first trained model.

The third trained model recording unit 274 records a third trained model. Specifically, the third trained model is a trained model that has been trained by using an already known training model and using a plurality of pieces of training data each of which is obtained in a different situation from that used for the first trained model and the second trained model and in each of which the driving information, the device information, and the site information that are used as input parameters and are associated with the intraoperative information that is used as an output parameter. In the second trained model, the trained model that has been generated by using the same machine learning technique as that used for the first trained model is used, and the same training model is used, except that training data to be trained and a weighting factor or the like in the intermediate layer are different from those used in the first trained model.

The control unit 280B includes a calculation unit 286B, instead of the calculation unit 286 included in the control unit 280A according to the above described second embodiment.

The calculation unit 286B calculates the accuracy of the intraoperative information on the basis of the plurality of pieces of intraoperative information estimated by the estimation unit 282 using each of the first trained model, the second trained model, and the third trained model.

### Process performed by assistance device

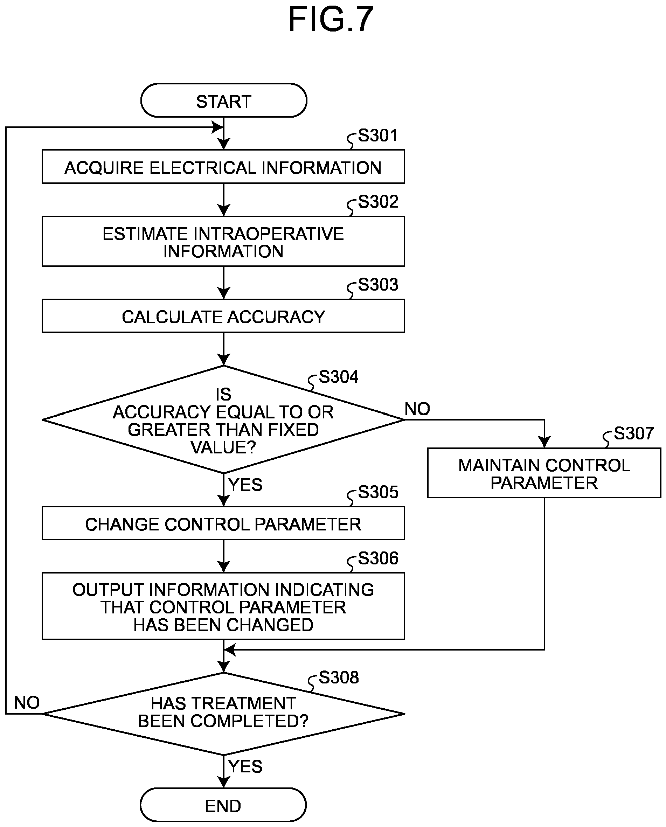

In the following, a process performed by the assistance device 200B will be described. FIG. 7 is a flowchart illustrating an outline of the process performed by the assistance device 200B. In FIG. 7, Step S301 and Step S305 to Step S308 correspond to Step S201 and Step S206 to Step S209, respectively, illustrated in FIG. 5 described above, and the same processes are performed at these steps; therefore, descriptions thereof in detail will be omitted. Accordingly, in the following description, each of the processes performed by the assistance device 200B at Step S302, Step S303, and Step S304 will be described.

At Step S302, the estimation unit 282 estimates the plurality of pieces of intraoperative information on the basis of the first trained model that is recorded by the first trained model recording unit 272, the second trained model that is recorded by the second trained model recording unit 273, the third trained model that is recorded by the third trained model recording unit 274, and the electrical information that has been acquired by the acquisition unit 281. Specifically, the estimation unit 282 inputs the electrical information that has been acquired by the acquisition unit 281 to the first trained model as the input parameter, and estimates the output parameter that is output from the first trained model as the intraoperative information (hereinafter, simply referred to as the "first intraoperative information") (the first estimation result). Similarly, the estimation unit 282 estimates the output parameter that is output from the second trained model as the intraoperative information (hereinafter, simply referred to as the "second intraoperative information") (the second estimation result). Furthermore, the estimation unit 282 estimates the output parameter that is output from the third trained model as the intraoperative information (hereinafter, simply referred to as the "third intraoperative information") (the third estimation result).

Subsequently, the calculation unit 286B calculates the accuracy of the intraoperative information on the basis of the plurality of pieces of intraoperative information that have been estimated by the estimation unit 282 (Step S303). Specifically, the calculation unit 286B calculates the accuracy of the intraoperative information on the basis of the accuracy included in each of the first intraoperative information, the second intraoperative information, and the third intraoperative information (Step S303). Specifically, the calculation unit 286B calculates, as the accuracy, the average value of the accuracy in an incision state of the target site included in each of the first intraoperative information, the second intraoperative information, and the third intraoperative information. Of course, the calculation unit 286B may calculate not only the average value of the accuracy, but also the median value or the mode value as the accuracy.

Subsequently, the determination unit 283 determines whether or not the accuracy calculated by the calculation unit 286B is equal to or greater than the fixed value (Step S304). If it is determined, by the determination unit 283, that the accuracy calculated by the calculation unit 286B is equal to or greater than the fixed value (Yes at Step S304), the assistance device 200B proceeds to Step S305. In contrast, if it is determined, by the determination unit 283, that the accuracy calculated by the calculation unit 286B is not equal to or greater than the fixed value (No at Step S304), the assistance device 200B proceeds to Step S307.

According to the above described third embodiment, if it is determined, by the determination unit 283, that the accuracy calculated by the calculation unit 286B on the basis of the accuracy included in the intraoperative information in each of the first trained model, the second trained model, and the third trained model is equal to or greater than the fixed value, the parameter control unit 284 changes the control parameter for controlling the treatment tool 110 performed by the treatment tool control device 120 on the basis of the intraoperative information that has been estimated by the estimation unit 282, and outputs the changed control parameter to the treatment tool control device 120. As a result of this, the operator is able to appropriately control the treatment tool 110 by using the control parameter having a high reliable estimation result of the first trained model.

### Fourth Embodiment

In the following, a fourth embodiment will be described. In the fourth embodiment, the accuracy is determined on the basis of two pieces of intraoperative information that have been estimated by using different trained models. Accordingly, in the following description, after a functional configuration of an assistance device according to the fourth embodiment has been described, the process performed by the assistance device according to the fourth embodiment will be described. Furthermore, components that are the same as those included in the assistance devices 200 and 200A, and 200B according to the first to the third embodiments, respectively, described above are assigned the same reference numerals and descriptions thereof in detail will be omitted.

### Functional configuration of assistance device

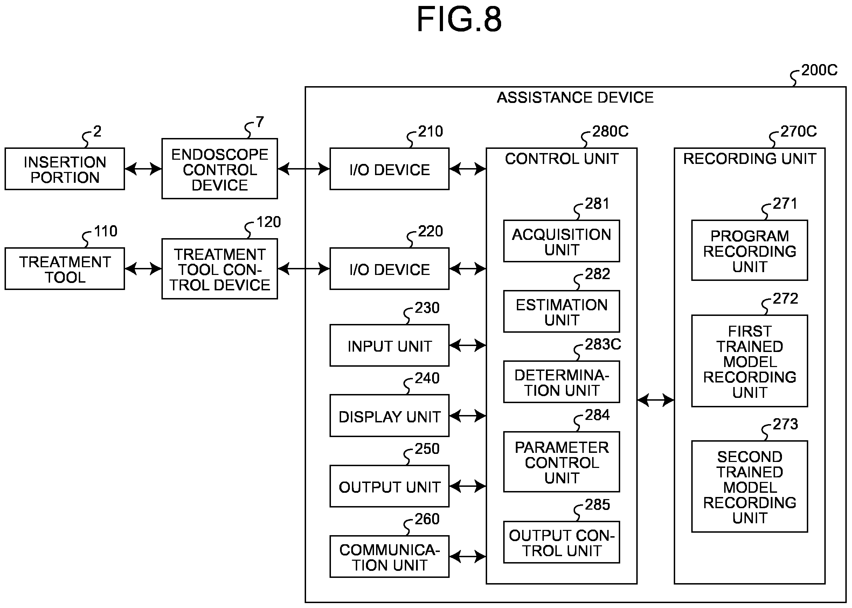

FIG. 8 is a block diagram illustrating the functional configuration of the assistance device according to the fourth embodiment. An assistance device 200C illustrated in FIG. 8 includes a recording unit 270C and a control unit 280C, instead of the recording unit 270 and the control unit 280 that are included in the assistance device 200 according to the above described first embodiment.

The recording unit 270C further includes the second trained model recording unit 273 according to the above described second embodiment, in addition to the functional configuration of the recording unit 270 according to the above described first embodiment.

The control unit 280C includes a determination unit 283C, instead of the determination unit 283 included in the control unit 280 according to the above described first embodiment.

The determination unit 283C determines whether or not the accuracy of each of the first intraoperative information and the second intraoperative information that have been estimated by the estimation unit 282 using the first trained model that is recorded by the first trained model recording unit 272 and the second trained model that is recorded by the second trained model recording unit 273, respectively, is equal to or greater than the fixed value.

### Process performed by assistance device

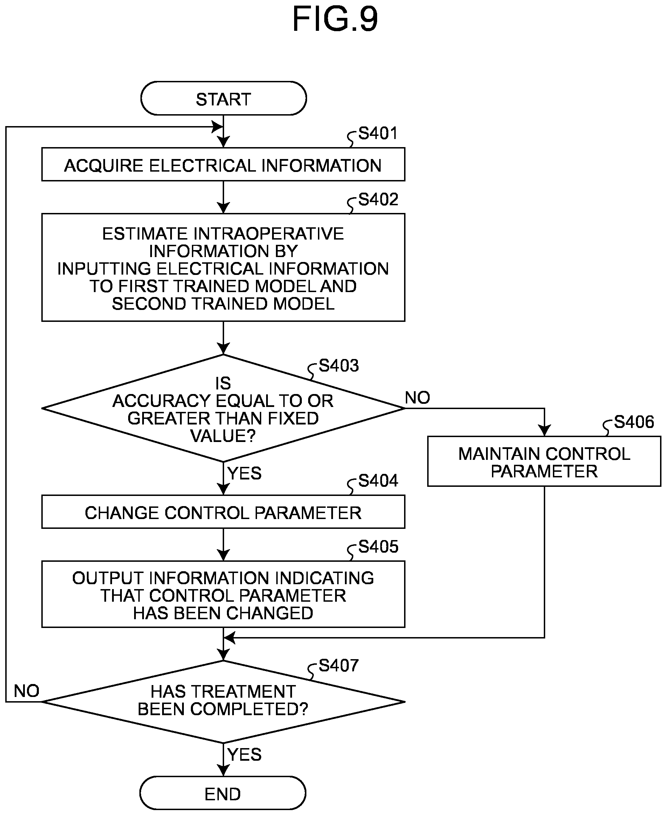

In the following, a process performed by the assistance device 200C will be described. FIG. 9 is a flowchart illustrating an outline of the process performed by the assistance device 200C. In FIG. 9, Step S401 and Step S404 to Step S406 correspond to Step S101 and Step S104 to Step S107, respectively, illustrated in FIG. 3 described above, and the same processes are performed at these steps; therefore, descriptions thereof in detail will be omitted. Accordingly, in the following description, each of the processes performed by the assistance device 200C at Step S402 and Step S404 will be described.

At Step S402, the estimation unit 282 estimates the intraoperative information indicated during an operation on the basis of the first trained model that is recorded by the first trained model recording unit 272, the second trained model that is recorded by the second trained model recording unit 273, and on the basis of the electrical information that has been acquired by the acquisition unit 281. Specifically, the estimation unit 282 inputs the electrical information that has been acquired by the acquisition unit 281 to the first trained model as the input parameter, and estimates the output parameter that is output from the first trained model as the first intraoperative information. Similarly, the estimation unit 282 inputs the electrical information that has been acquired by the acquisition unit 281 to the second trained model as the input parameter, and estimates the output parameter that is output from the second trained model as the second intraoperative information.

Subsequently, the determination unit 283C determines whether or not the accuracy of each of the first intraoperative information and the second intraoperative information estimated by the estimation unit 282 is equal to or greater than the fixed value (Step S403). If it is determined, by the determination unit 283, that the accuracy of each of the first intraoperative information and the second intraoperative information estimated by the estimation unit 282 is equal to or greater than the fixed value (Yes at Step S403), the assistance device 200C proceeds to Step S404. In contrast, if it is determined, by the determination unit 283, that the accuracy of each of the first intraoperative information and the second intraoperative information estimated by the estimation unit 282 is not equal to or greater than the fixed value (No at Step S403), the assistance device 200C proceeds to Step S405.

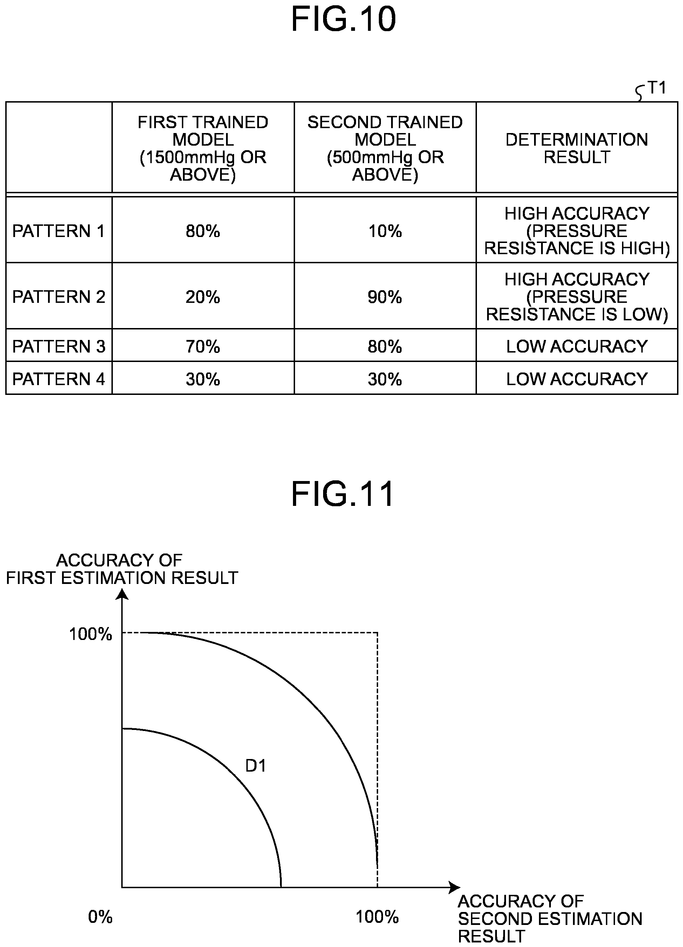

FIG. 10 is a diagram schematically illustrating determination content determined by the determination unit 283C. FIG. 11 is a diagram illustrating one example of a determination result determined by the determination unit 283C. In FIG. 11, the vertical axis indicates the accuracy (%) of the first estimation result that is the first intraoperative information, and the horizontal axis indicates the accuracy (%) of the second estimation result that is the second intraoperative information.

As illustrated in FIG. 11, the determination unit 283C determines whether or not the accuracy of each of the first intraoperative information and the second intraoperative information estimated by the estimation unit 282 is included in an area D1. For example, as indicated in the first row of the determination result table T1 illustrated in FIG. 10, in a case where the accuracy of the first intraoperative information is "80%", and the accuracy of the second intraoperative information is "10%" (the area D1 illustrated in FIG. 11), the determination unit 283C determines that the accuracy is high, and determines that the accuracy of each of the first intraoperative information and the second intraoperative information is equal to or greater than the fixed value. That is, the determination unit 283C determines that the pressure resistance value of the blood vessel is high on the basis of the accuracy of each of the first intraoperative information and the second intraoperative information.

Similarly, as indicated in the second row of the determination result table T1 illustrated in FIG. 10, in a case where the accuracy of the first intraoperative information is "20%", and the accuracy of the second intraoperative information is "90%" (the area D1 illustrated in FIG. 11), the determination unit 283C determines that the accuracy is high, and determines that the accuracy of each of the first intraoperative information and the second intraoperative information is equal to or greater than the fixed value. That is, the determination unit 283C determines that the pressure resistance value of the blood vessel is low on the basis of the accuracy of each of the first intraoperative information and the second intraoperative information.

In contrast, as indicated in the third row of the determination result table T1 illustrated in FIG. 10, in a case where the accuracy of the first intraoperative information is "70%", and the accuracy of the second intraoperative information is "80%" (outside the area D1 illustrated in FIG. 11), the determination unit 283C determines that the accuracy is low, and determines that the accuracy of each of the first intraoperative information and the second intraoperative information is not equal to or greater than the fixed value. Furthermore, as indicated in the fourth row of the determination result table T1 illustrated in FIG. 10, in a case where the accuracy of the first intraoperative information is "30%", and the accuracy of the second intraoperative information is "30%" (outside the area D1 illustrated in FIG. 11), the determination unit 283C determines that the accuracy is low, and determines that the accuracy of each of the first intraoperative information and the second intraoperative information is not equal to or greater than the fixed value.

According to the above described fourth embodiment, if it is determined, by the determination unit 283C, that accuracy calculated on the basis of the accuracy included in the intraoperative information obtained from each of the first trained model and the second trained model is equal to or greater than the fixed value, the parameter control unit 284 changes, on the basis of the intraoperative information estimated by the estimation unit 282, the control parameter for controlling the treatment tool 110 performed by the treatment tool control device 120, and outputs the changed control parameter to the treatment tool control device 120. As a result of this, the operator is able to appropriately control the treatment tool 110 by using the control parameter having a high reliable estimation result of the first trained model.

### Fifth Embodiment

In the following, a fifth embodiment will be described. In the fifth embodiment, the accuracy (deviation) of the electrical information that has been acquired by the acquisition unit 281 with respect to the training data that has been trained by the first trained model is equal to or greater than the fixed value, and, if the determination result is equal to or greater than the fixed value, the electrical information is input as an input parameter for the first trained model. Accordingly, in the following description, after a functional configuration of the assistance device according to the fifth embodiment has been described, a process performed by the assistance device according to the fifth embodiment will be described. Furthermore, components that are the same as those included in the assistance devices 200 and 200A to 200C according to the first to the fourth embodiments, respectively, described above are assigned the same reference numerals and descriptions thereof in detail will be omitted.

### Functional configuration of assistance device

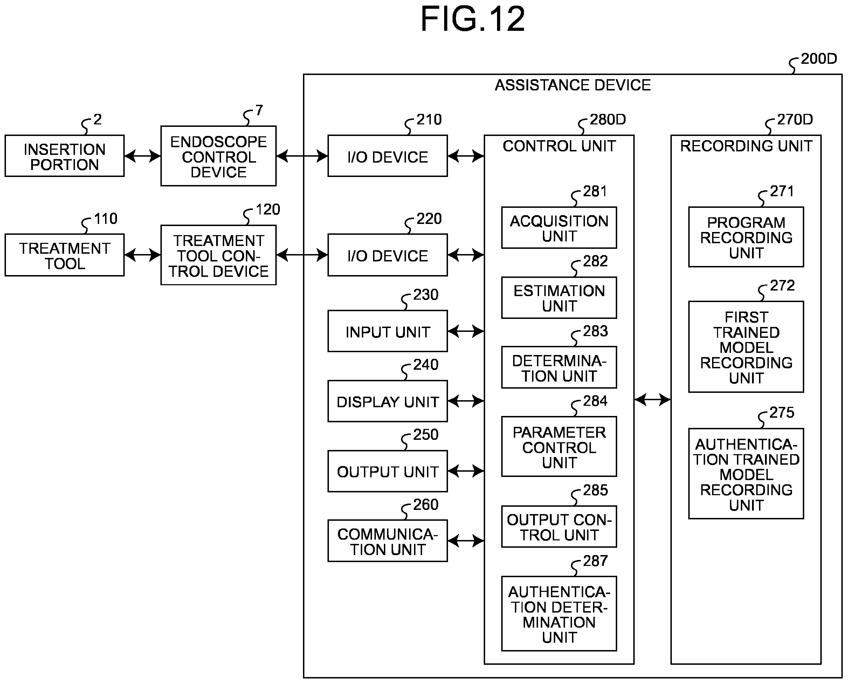

FIG. 12 is a block diagram illustrating the functional configuration of the assistance device according to the fifth embodiment. An assistance device 200D illustrated in FIG. 12 includes a recording unit 270D and a control unit 280D, instead of the recording unit 270 and the control unit 280 included in the assistance device 200 according to the above described first embodiment.

The recording unit 270D includes an authentication trained model recording unit 275, in addition to the functional configuration of the recording unit 270 according to the above described first embodiment.

The authentication trained model recording unit 275 records an authentication trained model. Specifically, the authentication trained model is a trained model that has been trained by using an already known training model and using a plurality of pieces of training data each of which is obtained in a different situation from that used for the first trained model and in each of which the driving information, the device information, and the site information that are used as input parameters and are associated with the accuracy that is related to deviation from the training data trained by the first trained model and that is used as an output parameter. In the authentication trained model, the trained model that has been generated by using the same machine learning technique as that used for the first trained model is used, and the same training model is used, except that the training data to be trained is different from those used in the first trained model.

The control unit 280D further includes an authentication determination unit 287, in addition to the control unit 280 according to the above described first embodiment.

The authentication determination unit 287 inputs the electrical information that has been acquired by the acquisition unit 281 to the authentication trained model that is recorded by the authentication trained model recording unit 275, estimates the accuracy of the electrical information as an estimation result, and determines whether or not the accuracy of this electrical information is equal to or greater than the fixed value.

### Process performed by assistance device

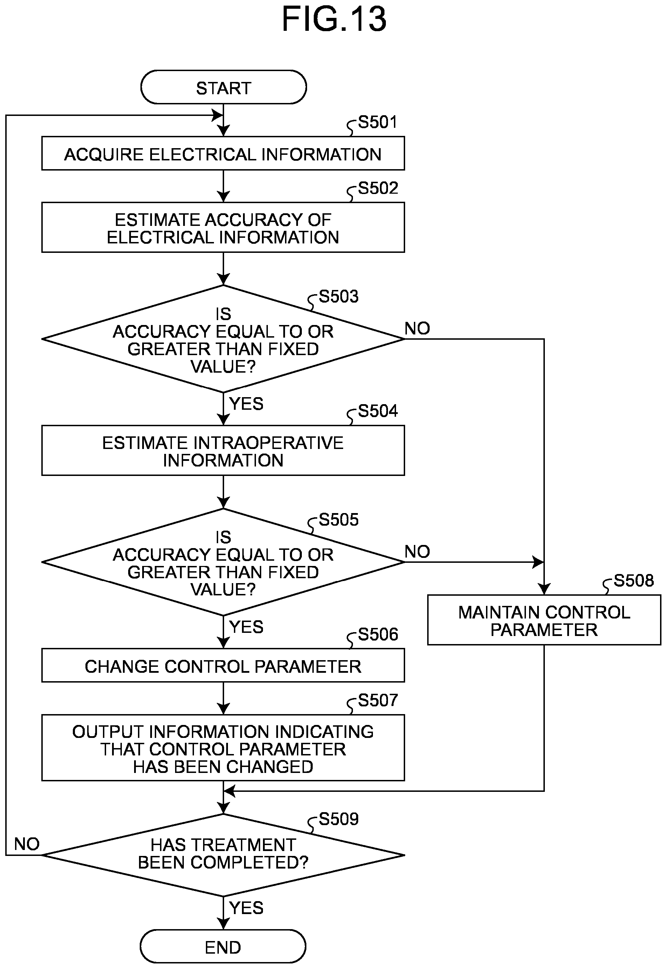

In the following, a process performed by the assistance device 200D will be described. FIG. 13 is a flowchart illustrating an outline of the process performed by the assistance device 200D. In FIG. 13, Step S501 and Step S504 to Step S509 correspond to Step S101 and Step S102 to Step S107, respectively, illustrated in FIG. 3 described above, and the same processes are performed at these steps; therefore, descriptions thereof in detail will be omitted. Accordingly, in the following description, each of the processes performed by the assistance device 200D at Step S502 and Step S503 will be described.

At Step S502, the authentication determination unit 287 inputs the electrical information that has been acquired by the acquisition unit 281 to the authentication trained model that is recorded by the authentication trained model recording unit 275 as the input parameter, and estimates the output parameter that is output from the authentication trained model as the accuracy of the electrical information indicating deviation from the training data that has been trained by the first trained model.

Subsequently, the authentication determination unit 287 determines whether or not the accuracy of the electrical information that has been estimated at Step S502 is equal to or greater than the fixed value (Step S503). If it is determined, by the authentication determination unit 287, that the accuracy of the electrical information that has been estimated at Step S502 is equal to or greater than the fixed value (Yes at Step S403), the assistance device 200D proceeds to Step S504. In contrast, if it is determined, by the authentication determination unit 287, that the accuracy of the electrical information that has been estimated at Step S502 is not equal to or greater than the fixed value (No at Step S403), the assistance device 200D proceeds to Step S508.

According to the above described fifth embodiment, in a case where it is determined, by the authentication determination unit 287, that the accuracy of the electrical information is equal to or greater than the fixed value, if it is determined, by the determination unit 283, that the accuracy that is included in the intraoperative information estimated by the estimation unit 282 is equal to or greater than the fixed value, the parameter control unit 284 changes, on the basis of the intraoperative information estimated by the estimation unit 282, the control parameter for controlling the treatment tool 110 performed by the treatment tool control device 120, and outputs the changed control parameter to the treatment tool control device 120. As a result of this, the operator is able to appropriately control the treatment tool 110.

### Sixth Embodiment

In the following, a sixth embodiment will be described. Conventionally, there are individual differences in target living tissue, and, in addition, a tissue state is influenced by the target tissue and a treatment environment (a grasping state (a diagonal grasp, a grasp at the distal end, unnecessary tissue is stuck, or a wet environment), so that it is difficult to select an optimum control parameter. Furthermore, in recent years, tissue identification and a sealed state are estimated by using the machine learning algorithm, but, there may be a case in which the estimated result is deviated from the trained model that is generated in advance in an actual use situation. Accordingly, in the sixth embodiment, it is estimated whether or not a scene (situation) is available for estimation in the first trained model, and, if the scene is available for the estimation in the first trained model, intraoperative information is estimated by the first trained model by inputting the electrical information to the first trained model. In the following description, after a functional configuration of the assistance device according to the sixth embodiment has been described, a process performed by the assistance device according to the sixth embodiment will be described. Furthermore, components that are the same as those included in the assistance devices 200 and 200A to 200D according to the first to the fifth embodiments described above are assigned the same reference numerals and descriptions thereof in detail will be omitted.

### Functional configuration of assistance device

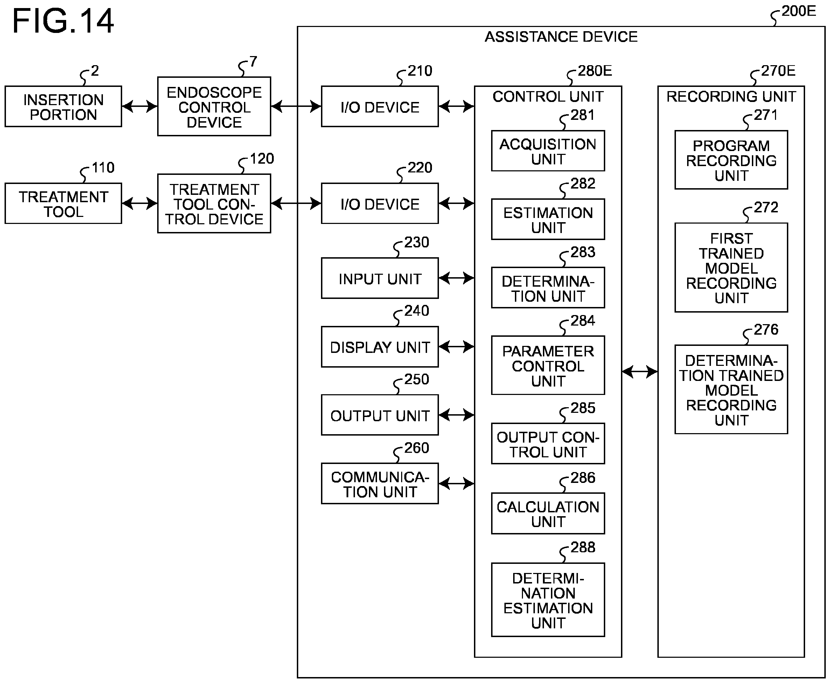

FIG. 14 is a block diagram illustrating the functional configuration of the assistance device according to the sixth embodiment. An assistance device 200E illustrated in FIG. 14 includes a recording unit 270E and a control unit 280E, instead of the recording unit 270 and the control unit 280A included in the assistance device 200A according to the above described second embodiment.

The recording unit 270E further includes a determination trained model recording unit 276, in addition to the functional configuration of the recording unit 270 according to the above described second embodiment.

The determination trained model recording unit 276 records a determination trained model. Specifically, the determination trained model is a trained model that has been trained by using an already known training model and using a plurality of pieces of training data each of which is obtained in a different situation from that used for the first trained model and in each of which the driving information, the device information, and the site information that are used as input parameters and are associated with association information that indicates whether or not the first trained model is available to be used and that is used as an output parameter. In the determination trained model, the trained model that has been generated by using the same machine learning technique as that used for the first trained model is used, and the same training model is used, except that training data to be trained is different from that used in the first trained model.

The control unit 280E further includes a determination estimation unit 288, in addition to the functional configuration of the control unit 280A according to the above described second embodiment.

The determination estimation unit 288 estimates, on the basis of the determination trained model that is recorded by the determination trained model recording unit 276 and the electrical information that has been acquired by the acquisition unit 281, the association information that indicates whether or not the first trained model is available to be used. Specifically, the determination estimation unit 288 inputs the electrical information to the determination trained model as an input parameter, and estimates the output parameter that is output from the determination trained model as the association information.

### Process performed by assistance device

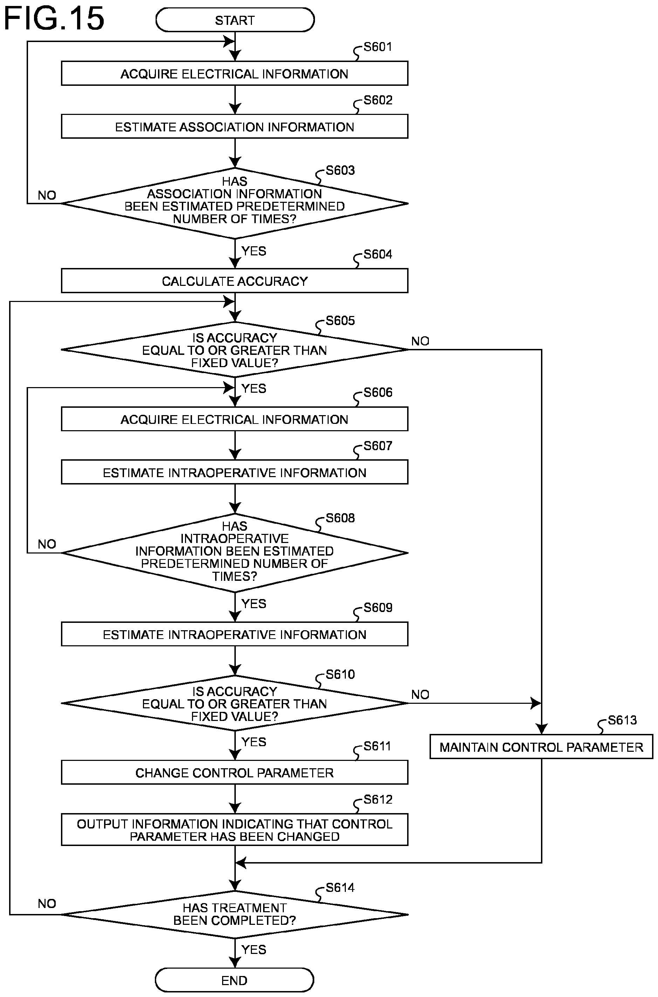

In the following, a process performed by the assistance device 200E will be described. FIG. 15 is a flowchart illustrating an outline of the process performed by the assistance device 200E. In FIG. 15, Step S606 to Step S613 correspond to Step S201 to Step S209, respectively, illustrated in FIG. 5 described above, and the same processes are performed at these steps; therefore, descriptions thereof in detail will be omitted. Accordingly, in the following description, each of the processes performed by the assistance device 200E at Step S601 to Step S605 will be described.

The acquisition unit 281 acquires the electrical information from the treatment tool control device 120 included in the treatment system 100 via the I/O device 220 (Step S601).

At Step S602, the determination estimation unit 288 estimates the association information indicating whether or not the first trained model is available to be used on the basis of the determination trained model that is recorded by the determination trained model recording unit 276 and on the basis of the electrical information that has been acquired by the acquisition unit 281 (Step S602). Specifically, the determination estimation unit 288 inputs the electrical information to the determination trained model as an input parameter, and estimates the output parameter that is output from the determination trained model as the association information.

The determination unit 283 determines whether or not the determination estimation unit 288 has performed the estimation of the association information a predetermined number of times (Step S603). Specifically, the determination unit 283 determines whether or not the determination estimation unit 288 has estimated the association information, for example, 10 or more times. If it is determined, by the determination unit 283, that the determination estimation unit 288 has performed the estimation of the association information the predetermined number of times (Yes at Step S603), the assistance device 200E proceeds to Step S604 that will be described later. In contrast, if it is determined, by the determination unit 283, that the determination estimation unit 288 has not performed the estimation of the association information the predetermined number of times (No at Step S603), the assistance device 200E returns to Step S601.

At Step S604, the calculation unit 286 calculates the accuracy of the association information on the basis of a plurality of pieces of association information estimated by the determination estimation unit 288 performed the predetermined number of times. Specifically, the calculation unit 286 calculates, as the accuracy of the association information, the average value of the accuracy of each of the plurality of pieces of association information estimated by the determination estimation unit 288 performed the predetermined number of times.

Subsequently, the determination unit 283 determines whether or not the accuracy calculated by the calculation unit 286 is equal to or greater than the fixed value (Step S605). If it is determined, by the determination unit 283, that the accuracy calculated by the calculation unit 286 is equal to or greater than the fixed value (Yes at Step S605), the assistance device 200E proceeds to Step S606. In contrast, if it is determined, by the determination unit 283, that the accuracy calculated by the calculation unit 286 is not equal to or greater than the fixed value (No at Step S605), the assistance device 200E proceeds to Step S613.

According to the above described sixth embodiment, in a case where it is estimated, by the determination unit 283, that the determination estimation unit 288 has performed estimation of the association information performed the predetermined number of times, if it is determined, by the determination unit 283, that the accuracy included in the intraoperative information estimated by the estimation unit 282 is equal to or greater than the fixed value, the parameter control unit 284 changes, on the basis of the intraoperative information that has been estimated by the estimation unit 282, the control parameter for controlling the treatment tool 110 performed by the treatment tool control device 120, and outputs the changed control parameter to the treatment tool control device 120. As a result of this, the operator is able to appropriately control the treatment tool 110.

### Seventh Embodiment

In the following, a seventh embodiment will be described. In the seventh embodiment, situation information indicated during an operation is estimated, and an optimum trained model is selected from a plurality of trained models on the basis of the obtained estimation result. Accordingly, in the following description, after a functional configuration of an assistance device according to the seventh embodiment has been described, a process performed by the assistance device according to the seventh embodiment will be described. Furthermore, components that are the same as those included in the assistance devices 200 and 200A to 200E according to the first to the sixth embodiments described above are assigned the same reference numerals and descriptions thereof in detail will be omitted.

### Functional configuration of assistance device

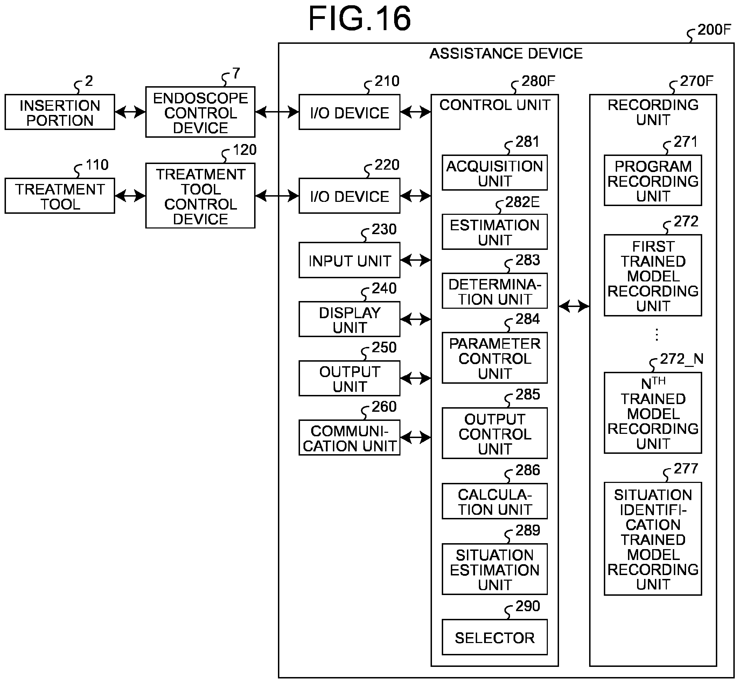

FIG. 16 is a block diagram illustrating the functional configuration of the assistance device according to the seventh embodiment. An assistance device 200F illustrated in FIG. 16 includes a recording unit 270F and a control unit 280F, instead of the recording unit 270 and the control unit 280A included in the assistance device 200A according to the above described second embodiment.

The recording unit 270F includes a plurality of N trained model recording units 272_N (N is an integer equal to or greater than 2) each of which records a trained model, and a situation identification trained model recording unit 277, in addition to the first trained model recording unit 272 included in the recording unit 270 according to the above described first embodiment.

The plurality of N trained model recording units 272_N record the respective trained models that have been obtained by training a plurality of pieces of respective training data acquired in different situations. Specifically, a second trained model recording unit 272_2 records a trained model that can be output and in which the electrical information is used as an input parameter and the intraoperative information including a control parameter for carotid artery treatment is used as an output parameter. A third trained model recording unit 272_3 records a trained model that can be output and in which the electrical information is used as an input parameter and the intraoperative information including a control parameter for oozing treatment is used as an output parameter. A fourth trained model recording unit 272_4 records a trained model that can be output and in which the electrical information is used as an input parameter and the intraoperative information including a control parameter for a diagonal grasp is used as an output parameter. In each of the plurality of N trained model recording units 272_N, the trained model that has been generated by using the same machine learning technique as that used for the first trained model is used, and the same training model is used, except that training data to be trained is different from that used in the first trained model. Moreover, in the seventh embodiment, a case in which the first to the fourth trained models are provided will be described as one example, but the example is not limited to this, and the number of trained models may be changed as needed.

The situation identification trained model recording unit 277 records a situation identification trained model. Specifically, the situation identification trained model is a trained model that has been trained by using an already known training model and using a plurality of pieces of training data each of which is obtained in a different situation from that used for the first trained model and in each of which the driving information, the device information, and the site information that are used as input parameters and are associated with the situation identification that is used to select an optimum trained model for an intraoperative from among the plurality of N trained models and that is used as an output parameter. In the situation identification trained model, the trained model that has been generated by using the same machine learning technique as that used for the first trained model is used, and the same training model is used, except that training data to be trained is different from that used in the first trained model.

The control unit 280F further includes a situation estimation unit 289 and a selector 290, in addition to the functional configuration of the control unit 280F according to the above described second embodiment.

The situation estimation unit 289 estimates the situation information related to the situation of the target site on the basis of the situation identification trained model that is recorded by the situation identification trained model recording unit 277 and on the basis of the electrical information that has been acquired by the acquisition unit 281. Specifically, an estimation unit 282F inputs the electrical information to the situation identification trained model as an input parameter, and estimates the output parameter that is output from the situation identification trained model as the situation information.

The selector 290 selects, on the basis of the situation information estimated by the situation estimation unit 289, an optimum trained model suitable for the current situation of the target site from among the first trained model recording unit 272 to the N^{th} trained model recording unit 272_N.

### Process performed by assistance device

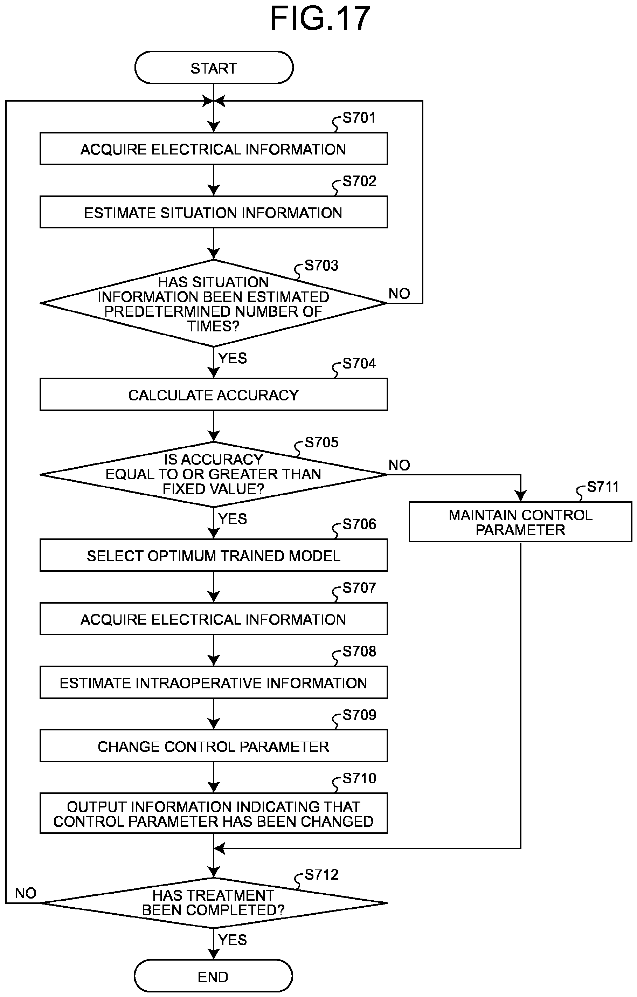

In the following, a process performed by the assistance device 200F will be described. FIG. 17 is a flowchart illustrating an outline of the process performed by the assistance device 200F. In FIG. 17, Step S701 and Step S709 to Step S712 correspond to Step S101 and Step S104 to Step S106, respectively, illustrated in FIG. 3 described above, and the same processes are performed at these steps; therefore, descriptions thereof in detail will be omitted. Accordingly, in the following description, each of the processes performed by the assistance device 200F at Step S702 and Step S708 will be described.

At Step S702, the estimation unit 282F estimates the situation information that is related to the site indicated during an operation on the basis of the situation identification trained model that is recorded by the situation identification trained model recording unit 277 and on the basis of the electrical information that has been acquired by the acquisition unit 281. Specifically, the estimation unit 282F estimates the situation information by inputting the electrical information to the situation identification trained model as an input parameter, and causing the situation identification trained model to output the situation information as an output parameter.

Subsequently, the determination unit 283 determines whether or not the estimation unit 282F has performed estimation of the situation information a predetermined number of times (Step S703). Specifically, the determination unit 283 determines whether or not the estimation unit 282F has performed estimation of the situation information five or more times. If it is determined, by the determination unit 283, that the estimation unit 282 has performed estimation of the situation information a predetermined number of times (Yes at Step S703), the assistance device 200F proceeds to Step S304 that will be described later. In contrast, if it is determined, by the determination unit 283, that the estimation unit 282 has not performed estimation of the situation information the predetermined number of times (No at Step S203), the assistance device 200F returns to Step S701.

At Step S704, the calculation unit 286 calculates the accuracy of the situation information on the basis of a plurality of pieces of situation information estimated the predetermined number of times by the estimation unit 282F. Specifically, the calculation unit 286 calculates, as the accuracy, the number of times of estimation of each of the scenes included in the associated pieces of situation information indicated by the estimation results of the estimation performed 10 or more times by the estimation unit 282F. Of course, in addition to the number of scenes included in the associated pieces of situation information indicated by the estimation results of the estimation performed 10 or more times by the estimation unit 282F, the calculation unit 286 may calculate, as the accuracy, the number of times of estimation of the tissue type, the number of times of estimation of the type of a blood vessel, the number of times of estimation of the size of the blood vessel, the number of times of estimation of the type of treatment, the number of times of estimation of sealing performed by treatment, the number of times of estimation of presence or absence of bleeding, the number of times of estimation of a grasping state of the treatment tool 110 (energy device), the number of times of estimation of surrounding environment (for example, an amount of connective tissue or an amount of moisture) of the target site, or the calculation unit 286 may calculate the median value or the mode value as the accuracy.

Subsequently, the determination unit 283 determines whether or not the accuracy calculated by the calculation unit 286 is equal to or greater than the fixed value (Step S705). If it is determined, by the determination unit 283, that the accuracy calculated by the calculation unit 286 is equal to or greater than the fixed value (Yes at Step S705), the assistance device 200F proceeds to Step S706. In contrast, if it is determined, by the determination unit 283, that the accuracy calculated by the calculation unit 286 is not equal to or greater than the fixed value (No at Step S705), the assistance device 200F proceeds to Step S711.

At Step S706, the selector 290 selects, on the basis of the situation information estimated by the situation estimation unit 289, an optimum trained model suitable for the current situation of the target site from among the plurality of trained models that are recorded by the plurality of respective N trained model recording units 272_N.

Subsequently, the acquisition unit 281 acquires the electrical information from the treatment tool control device 120 (Step S707).

Subsequently, the estimation unit 282 inputs the electrical information that has been acquired by the acquisition unit 281 to the optimum trained model that has been selected by the selector 290, and estimates the output parameter that is output from the optimum trained model as the intraoperative information including the control parameter (Step S708).

According to the above described seventh embodiment, the estimation unit 282 inputs the electrical information acquired by the acquisition unit 281 to the optimum trained model selected by the selector 290, and estimates the intraoperative information including the control parameter by causing the optimum trained model to output the result. The parameter control unit 284 changes, on the basis of the intraoperative information that has been estimated by the estimation unit 282, the control parameter for controlling the treatment tool 110 performed by the treatment tool control device 120, and outputs the changed control parameter to the treatment tool control device 120. As a result of this, the operator is able to appropriately control the treatment tool 110.

Furthermore, according to the seventh embodiment, the selector 290 selects the optimum trained model, and the estimation unit 282 estimates and changes the control parameter. As a result of this, it is possible to more precisely perform determination and adjustment of the control parameter as compared with a case in which the control parameter is simply changed by using a trained model, and, in addition, it is advantageous in terms of a processing speed or the like.

### Eighth Embodiment

In the following, an eighth embodiment will be described. Conventionally, if the treatment tool 110 continuously outputs energy as an empty output while maintaining the grasping state after the completion of tissue incision of the target site, this may lead to an excessive rise in temperature at the distal end of the treatment tool 110 or lead to damage of the vibration transmission member 119. In order to prevent this, there is a known technology for detecting the completion of tissue incision on the basis of variations in load applied to the treatment tool 110 and automatically stopping the output. However, there may be a case in which a detection rate of the completion of the tissue incision of the target site is decreased depending on a grasp position of tissue grasped by the treatment tool 110 (for example, in a case where the target site is grasped at the distal end of the vibration transmission member 119 included in the treatment tool 110). Accordingly, in the eighth embodiment, a grasping situation of the target site grasped by the treatment tool 110 is estimated, and a control parameter is estimated in accordance with the estimated grasping situation. In the following description, after a functional configuration of an assistance device according to the eighth embodiment has been described, a process performed by the assistance device according to the eighth embodiment will be described. Furthermore, components that are the same as those included in the assistance devices 200 and 200A to 200F according to the first to the seventh embodiments described above are assigned the same reference numerals and descriptions thereof in detail will be omitted.

### Functional configuration of assistance device

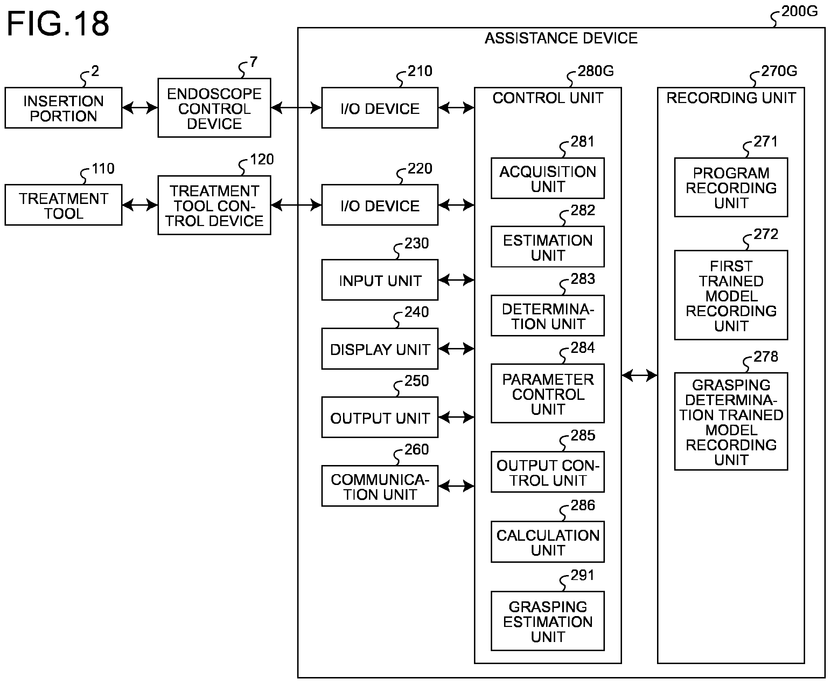

FIG. 18 is a block diagram illustrating the functional configuration of the assistance device according to the eighth embodiment. An assistance device 200G illustrated in FIG. 18 includes a recording unit 270G and a control unit 280G, instead of the recording unit 270 and the control unit 280A included in the assistance device 200A according to the above described second embodiment.

The recording unit 270G further includes a grasping determination trained model recording unit 278, in addition to the functional configuration of the recording unit 270 according to the above described second embodiment.

The grasping determination trained model recording unit 278 records a grasping determination trained model. Specifically, the grasping determination trained model is a trained model that has been trained by using an already known training model and using a plurality of pieces of training data each of which is obtained in a different situation from that used for the first trained model and in each of which the driving information, the device information, and the site information that are used as input parameters and are associated with both of the target site and the grasping information that indicate the state of a grasp performed by the treatment tool 110 and that are used as output parameters. In the grasping determination trained model, the trained model that has been generated by using the same machine learning technique as that used for the first trained model is used, and the same training model is used, except that training data to be trained is different from that used in the first trained model.

The control unit 280G further includes a grasping estimation unit 291, in addition to the functional configuration of the control unit 280A according to the above described second embodiment.

The grasping estimation unit 291 inputs the electrical information, as the input parameter, that has been acquired by the acquisition unit 281 to the grasping determination trained model that is recorded by the grasping determination trained model recording unit 278, and estimates the output parameter, as the grasping information, that is output from the grasping determination trained model.

### Process performed by assistance device

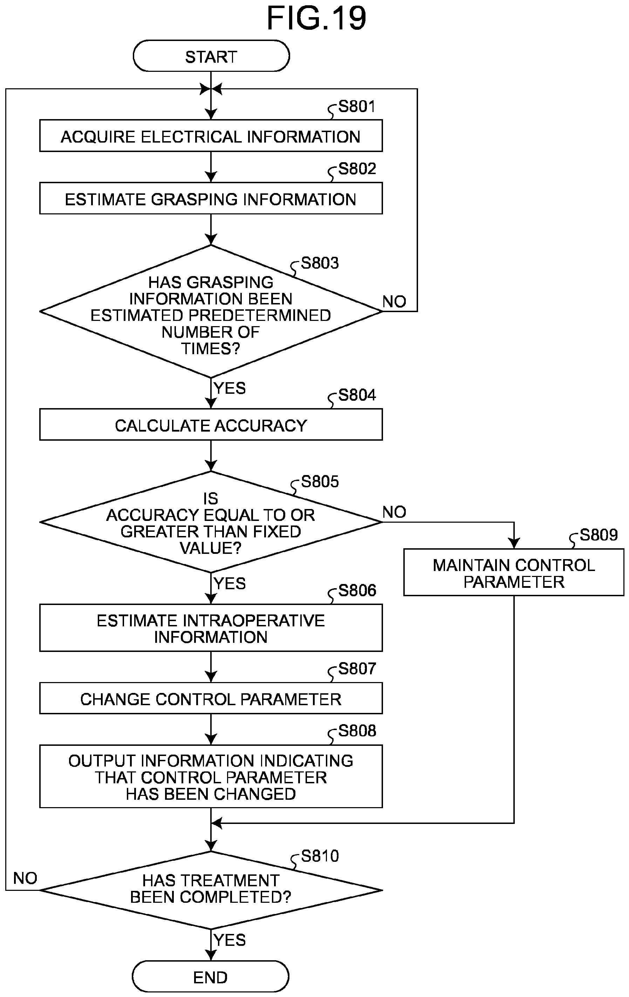

In the following, a process performed by the assistance device 200G will be described. FIG. 19 is a flowchart illustrating an outline of the process performed by the assistance device 200G. Step S801 and Step S806 to Step S810 correspond to Step S101, Step S102, Step S104 to Step S107 described above, respectively, illustrated in FIG. 3 described above, and the same processes are performed at these steps; therefore, descriptions thereof in detail will be omitted. Accordingly, in the following description, each of the processes performed by the assistance device 200G at Step S802 to Step S805 will be described.

At Step S802, the grasping estimation unit 291 estimates grasping information related to the target site grasped by the treatment tool 110 on the basis of the grasping situation trained model that is recorded by the grasping determination trained model recording unit 278 and on the basis of the electrical information that has been acquired by the acquisition unit 281. Specifically, an estimation unit 282G inputs the electrical information to the grasping situation trained model as an input parameter, and estimates the grasping information by causing the grasping situation trained model to output the grasping information as an output parameter.

Subsequently, the determination unit 283 determines whether or not the grasping estimation unit 291 has performed estimation of the grasping information a predetermined number of times (Step S803). If it is determined, by the determination unit 283, that the grasping estimation unit 291 has performed estimation of the grasping information the predetermined number of times (Yes at Step S803), the assistance device 200G proceeds to Step S804 that will be described later. In contrast, if it is determined, by the determination unit 283, that the grasping estimation unit 291 has not performed estimation of the grasping information the predetermined number of times (No at Step S803), the assistance device 200G returns to Step S801.

At Step S804, the calculation unit 286 calculates the accuracy of the grasping information on the basis of a plurality of pieces of grasping information that have been estimated the predetermined number of times by the grasping estimation unit 291. Specifically, the calculation unit 286 calculates, as the accuracy, the average value of the accuracy of the grasping state included in the respective pieces of grasping information indicated by the associated estimation results obtained 10 times obtained by the grasping estimation unit 291. Of course, the calculation unit 286 may calculate, as the accuracy, a grasped area or a contact area of the target site included in each of the pieces of grasping information on the associated estimation results obtained 10 times by the grasping estimation unit 291, or the calculation unit 286 may calculate the median value or the mode value as the accuracy.

Subsequently, the determination unit 283 determines whether or not the accuracy calculated by the calculation unit 286 is equal to or greater than the fixed value (Step S805). If it is determined, by the determination unit 283, that the accuracy calculated by the calculation unit 286 is equal to or greater than the fixed value (Yes at Step S805), the assistance device 200G proceeds to Step S806. In contrast, if it is determined, by the determination unit 283, that the accuracy calculated by the calculation unit 286 is not equal to or greater than the fixed value (No at Step S805), the assistance device 200G proceeds to Step S809.

According to the above described eighth embodiment, if it is determined, by the determination unit 283, that the accuracy that has been calculated by the calculation unit 286 and that is related to the grasping information indicated by the estimation result obtained by the grasping estimation unit 291 is equal to or greater than the fixed value, the parameter control unit 284 changes, on the basis of the intraoperative information estimated by the estimation unit 282, the control parameter for controlling the treatment tool 110 performed by the treatment tool control device 120, and outputs the changed control parameter to the treatment tool control device 120. As a result of this, the operator is able to appropriately control the treatment tool 110 by using the control parameter having a high reliable estimation result of the first trained model.

### Ninth Embodiment

In the following, a ninth embodiment will be described. Conventionally, the energy device that performs surgical treatment by using high frequency energy performs control of output electric power, a voltage, and the like on the basis of a detected electrical signal. In a case where this type of energy device is used for sealing a blood vessel, the energy device controls an output of the output electric power, the output voltage, and the like by detecting a tissue state in order to obtain a suitable sealed state. Furthermore, in a conventional technology, an output of the energy device is stopped by performing tissue identification using two sizes (an S size and an L size), and satisfying each of the set end resistance and the minimum output time. As a result of this, in the conventional technology, control to be performed on a blood vessel for each size of the target site is not optimized due to a small number of categories of the tissue identification, and thus, a control parameter is fixed to one of these two sizes. As a result of this, due to tissue misidentification and the small number of categories of the tissue identification, a technology that enables treatment performed by an optimum control parameter for the energy device in each size of the blood vessel is needed. Accordingly, in the ninth embodiment, treatment is performed by the energy device using an optimum control parameter for the size of the target site by deciding a control parameter for treatment performed by the treatment system 100, and then, by causing the trained model to estimate the intraoperative information by inputting the electrical information, and changing a detailed control parameter on the basis of the intraoperative information. In the following description, after a functional configuration of an assistance device according to the ninth embodiment has been described, a process performed by the assistance device according to the ninth embodiment will be described. Furthermore, components that are the same as those included in the assistance devices 200 and 200A to 200G according to the first to the eighth embodiments described above are assigned the same reference numerals and descriptions thereof in detail will be omitted.

### Functional configuration of assistance device

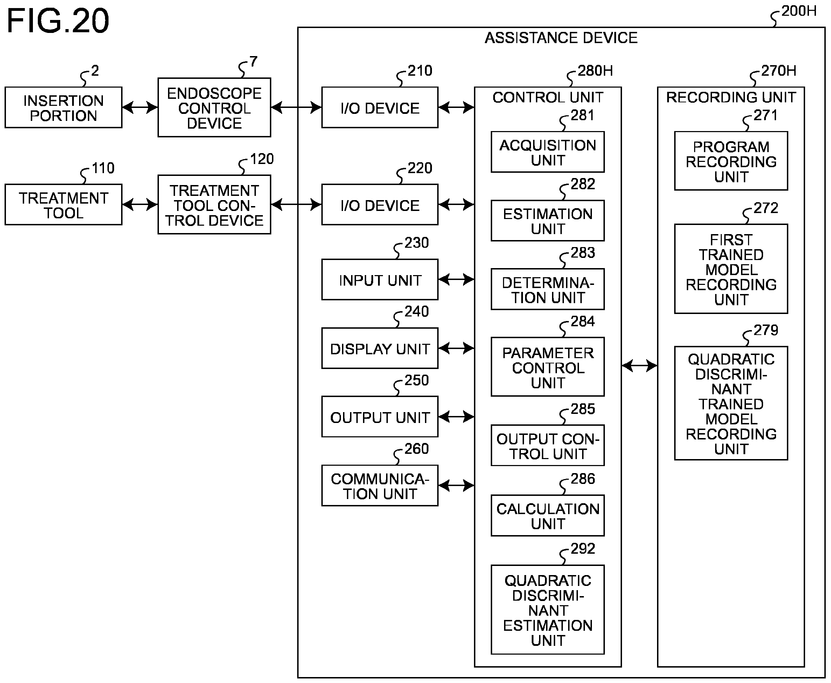

FIG. 20 is a block diagram illustrating the functional configuration of the assistance device according to the ninth embodiment. An assistance device 200H illustrated in FIG. 20 includes a recording unit 270H and a control unit 280H, instead of the recording unit 270 and the control unit 280A included in the assistance device 200A according to the above described second embodiment.

The recording unit 270H further includes a quadratic discriminant trained model recording unit 279, in addition to the functional configuration of the recording unit 270 according to the above described second embodiment.

The quadratic discriminant trained model recording unit 279 records a quadratic discriminant trained model. Specifically, the quadratic discriminant trained model is a trained model that has been trained by using an already known training model and using a plurality of pieces of training data each of which is obtained in a different situation from that used for the first trained model and in each of which the driving information, the device information, and the site information that are used as input parameters and are associated with quadratic discriminant information that includes the size of the target site, a blood vessel sealed state, a pressure resistance value of the blood vessel, and the like and that is used as an output parameter. In the quadratic discriminant trained model, the trained model that has been generated by using the same machine learning technique as that used for the first trained model is used, and the same training model is used, except that training data to be trained is different from that used in the first trained model.

The control unit 280H further includes a quadratic discriminant estimation unit 292, in addition to the functional configuration of the control unit 280A according to the above described second embodiment.

The quadratic discriminant estimation unit 292 decides a control parameter for performing treatment by the treatment system 100 on the basis of the electrical information that has been acquired by the acquisition unit 281 and on the basis of the quadratic discriminant trained model that is recorded by the quadratic discriminant trained model recording unit 279, and then, estimates the quadratic discriminant information related to the target site that is in a state before the intraoperative information used for performing treatment on the target site is estimated. Specifically, the quadratic discriminant estimation unit 292 inputs the electrical information acquired by the acquisition unit 281 to the quadratic discriminant trained model as an input parameter, and estimates the output parameter that is output from the quadratic discriminant trained model as the quadratic discriminant information.

### Process performed by assistance device

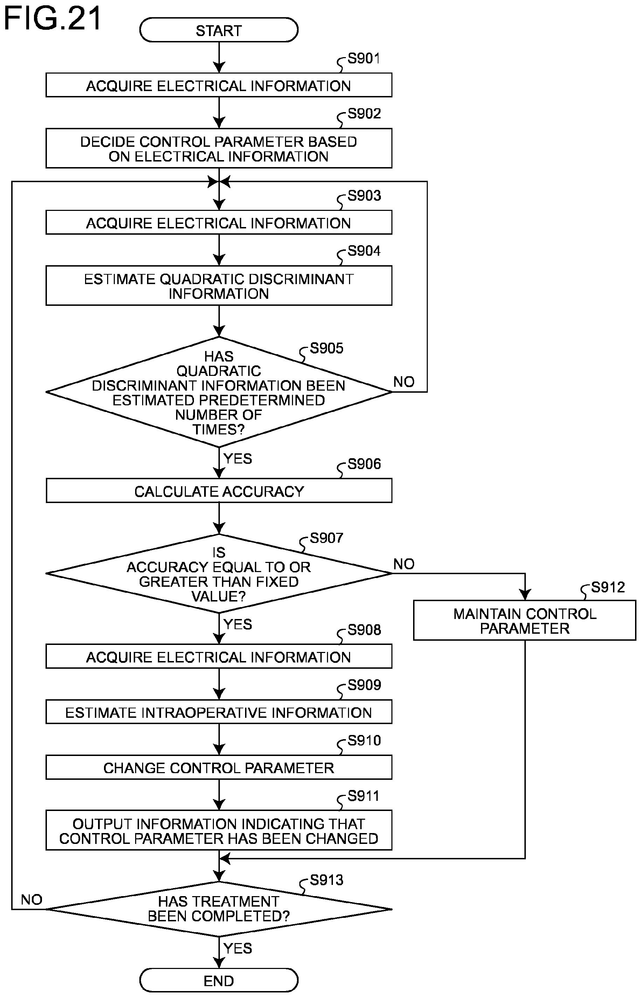

In the following, the process performed by the assistance device 200H will be described. FIG. 21 is a flowchart illustrating an outline of the process performed by the assistance device 200H.

As illustrated in FIG. 21, first, the acquisition unit 281 acquires the electrical information from the treatment tool control device 120 included in the treatment system 100 (Step S901).

Subsequently, the parameter control unit 284 decides, on the basis of the electrical information acquired by the acquisition unit 281, the control parameter for performing treatment on the target site by the treatment tool 110 (Step S902). In this case, the parameter control unit 284 decides the control parameter suitable for the size of the target site on the basis of at least one or more of a voltage, an electric current, electric power suitable for the voltage and the electric current, impedance, and a phase that are included in the electrical information acquired by the acquisition unit 281, and then outputs the decided control parameter to the treatment tool control device 120. Specifically, the parameter control unit 284 determines whether or not the size of the target site is equal to or greater than a predetermined value on the basis of at least one or more of a voltage, an electric current, electric power suitable for the voltage and the electric current, impedance, and a phase, and, if it is determined that the target site is greater than the predetermined value, the parameter control unit 284 decides the control parameter that is used in the case where the size of the target site is greater than the predetermined value (L size). In contrast, if the parameter control unit 284 determines that the size of the target site is not greater than the predetermined value (S size), the parameter control unit 284 decides the control parameter that is used in the case where the size of the target site is not greater than the predetermined value (S size). Here, the control parameter is an output voltage that is output to the target site by the treatment tool 110, an output electric current, output time, and an end resistance value.

Subsequently, the acquisition unit 281 acquires the electrical information from the treatment tool control device 120 (Step S903).

After that, the quadratic discriminant estimation unit 292 estimates the quadratic discriminant information on the basis of the quadratic discriminant trained model that is recorded by the quadratic discriminant trained model recording unit 279 and on the basis of the electrical information that has been acquired by the acquisition unit 281 (Step S904). Specifically, the quadratic discriminant estimation unit 292 inputs the electrical information acquired by the acquisition unit 281 to the quadratic discriminant trained model as an input parameter, and estimates the output parameter that is output from the quadratic discriminant trained model as the quadratic discriminant information.

After that, the determination unit 283 determines whether or not the quadratic discriminant estimation unit 292 has performed estimation of the quadratic discriminant information a predetermined number of times (Step S905). If it is determined, by the determination unit 283, that the quadratic discriminant estimation unit 292 has performed estimation of the quadratic discriminant information the predetermined number of times (Yes at Step S905), the assistance device 200H proceeds to Step S906 that will be described later. In contrast, if it is determined, by the determination unit 283, that the quadratic discriminant estimation unit 292 has not performed estimation of the quadratic discriminant information the predetermined number of times (No at Step S905), the assistance device 200H returns to Step S903.

At Step S906, the calculation unit 286 calculates the accuracy of the quadratic discriminant information on the basis of the plurality of pieces of quadratic discriminant information estimated the predetermined number of times by the quadratic discriminant estimation unit 292. Specifically, the calculation unit 286 calculates, as the accuracy, the number of times of estimation of each of the sizes (for example, 7 mm, 6 mm, 5 mm, and 4 mm) of the target site included in the associated pieces of quadratic discriminant information indicated by the estimation results of the estimation performed 10 or more times by the quadratic discriminant estimation unit 292. Of course, in addition to the number of times of estimation of each of the sizes of the target site included in the associated pieces of quadratic discriminant information indicated by the estimation results of the estimation performed the predetermined number of times by the quadratic discriminant estimation unit 292, the calculation unit 286 may calculate, as the accuracy, the number of times of estimation of the tissue type, the number of times of estimation of the type of a blood vessel, the number of times of estimation of the size of the blood vessel, the number of times of estimation of the type of treatment, the number of times of estimation of a sealing state generated by treatment, the number of times of estimation of presence or absence of bleeding, the number of times of estimation of a grasping state of the treatment tool 110 (energy device), the number of times of estimation of surrounding environment (for example, an amount of connective tissue or an amount of moisture) of the target site, or the calculation unit 286 may calculate the median value or the mode value as the accuracy.

Subsequently, the determination unit 283 determines whether or not the accuracy calculated by the calculation unit 286 is equal to or greater than the fixed value (Step S907). If it is determined, by the determination unit 283, that the accuracy calculated by the calculation unit 286 is equal to or greater than the fixed value (Yes at Step S907), the assistance device 200H proceeds to Step S908. In contrast, if it is determined, by the determination unit 283, that the accuracy calculated by the calculation unit 286 is not equal to or greater than the fixed value (No at Step S907), the assistance device 200H proceeds to Step S912.

At Step S908, the acquisition unit 281 acquires the electrical information from the treatment tool control device 120 included in the treatment system 100.

Subsequently, the estimation unit 282 estimates the intraoperative information indicated during an operation on the basis of the first trained model, the electrical information that has been acquired by the acquisition unit 281, and the quadratic discriminant information that has been estimated by the quadratic discriminant estimation unit 292 (Step S909).

Specifically, the estimation unit 282 inputs the electrical information acquired by the acquisition unit 281 and the quadratic discriminant information estimated by the quadratic discriminant estimation unit 292 to the first trained model as input parameters, and estimates the intraoperative information by allowing the first trained model to output the intraoperative information as the output parameter.

The parameter control unit 284 changes the control parameter for controlling the treatment tool 110 performed by the treatment tool control device 120 to the control parameter that is included in the intraoperative information estimated by the estimation unit 282, and outputs the changed control parameter (Step S910). For example, the parameter control unit 284 outputs, to the treatment tool control device 120, the control parameter for the output voltage that is supplied to the treatment tool 110 by the treatment tool control device 120 and that is increased or decreased. Of course, the parameter control unit 284 may stop the output voltage that is supplied to the treatment tool 110 by the treatment tool control device 120. As a result of this, the treatment tool control device 120 is able to supply, to the treatment tool 110, the high frequency energy signal and the ultrasound energy signal for performing optimum treatment on the target site that is being subjected to treatment by the treatment tool 110. As a result of this, the operator is able to perform optimum treatment on the target site by using the treatment tool 110.

Subsequently, the output control unit 285 outputs information indicating that the control parameter has been changed to the display unit 240 or the output unit 250 (Step S911). After the process performed at Step S911, the assistance device 200H proceeds to Step S913.

At Step S912, the parameter control unit 284 maintains the control parameter that has been decided at Step S902. As a result of this, it is possible to prevent the control parameter from being automatically changed in a low reliable state of the estimation result when the accuracy of the estimation result estimated by the estimation unit 282 is less than the fixed value. After the process performed at Step S912, the assistance device 200H proceeds to Step S913 that will be described later.

Subsequently, the determination unit 283 determines whether or not the operator has completed treatment performed on the subject (Step S913). If it is determined, by the determination unit 283, that the operator has completed treatment performed on the subject (Yes at Step S913), the assistance device 200H ends this process. In contrast, if it is determined, by the determination unit 283, that the operator has not completed treatment performed on the subject (No at Step S913), the assistance device 200H returns to Step S903 described above.

According to the above described ninth embodiment, the treatment tool control device 120 is able to supply, to the treatment tool 110, the high frequency energy signal and the ultrasound energy signal for performing optimum treatment on the target site that is being subjected to treatment by the treatment tool 110. As a result of this, the operator is able to perform optimum treatment on the target site by using the treatment tool 110.

Furthermore, according to the ninth embodiment, the estimation unit 282 estimates the intraoperative information indicated during an operation on the basis of the first trained model, the electrical information that has been acquired by the acquisition unit 281, and the quadratic discriminant information that has been estimated by the quadratic discriminant estimation unit 292, so that it is possible to perform precise and optimum control on the blood vessel size in accordance with each of the plurality of sizes, in addition to the only two sizes by using the estimation result obtained from machine learning.

### First modification of the ninth embodiment

In the following, a first modification of the ninth embodiment will be described. The first modification of the ninth embodiment has the same configuration as that of the assistance device 200H according to the above described ninth embodiment, but the process to be performed is different. Accordingly, in the following description, a process performed by an assistance device according to the first modification of the ninth embodiment will be described. Furthermore, components that are the same as those included in the assistance device 200H according to the above described ninth embodiment is assigned the same reference numerals and descriptions thereof in detail will be omitted.

### Process performed by assistance device

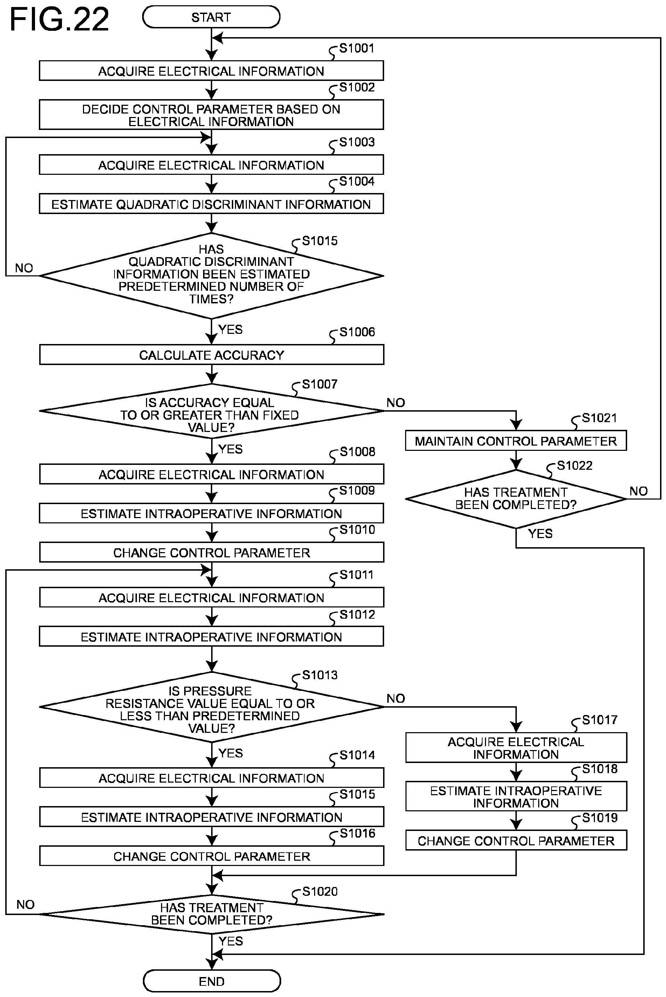

FIG. 22 is a flowchart illustrating an outline of the process performed by the assistance device 200H according to the first modification of the ninth embodiment. In FIG. 22, Step S1001 to Step S1010 correspond to Step S901 to Step S910, respectively, illustrated in FIG. 22 described above, and the same processes are performed at these steps; therefore, descriptions thereof in detail will be omitted. Accordingly, in the following description, each of the processes performed by at Step S1011 and the subsequent steps will be described.

At Step S1011, the acquisition unit 281 acquires the electrical information from the treatment tool control device 120 included in the treatment system 100.

Subsequently, the estimation unit 282 estimates the intraoperative information indicated during an operation on the basis of the first trained model, the electrical information that has been acquired by the acquisition unit 281, and the quadratic discriminant information that has been estimated by the quadratic discriminant estimation unit 292 (Step S1012).

After that, the determination unit 283 determines whether or not the pressure resistance value of the blood vessel included in the intraoperative information estimated by the estimation unit 282 is equal to or less than a predetermined value (Step S1013). For example, the determination unit 283 determines whether or not the pressure resistance value of the blood vessel included in the intraoperative information estimated by the estimation unit 282 is equal to or less than 1500 mm Hg. If it is determined, by the determination unit 283, that the pressure resistance value of the blood vessel included in the intraoperative information estimated by the estimation unit 282 is equal to or less than the predetermined value (Yes at Step S1013), the assistance device 200H proceeds to Step S1014 that will be described later. In contrast, if it is determined, by the determination unit 283, that the pressure resistance value of the blood vessel included in the intraoperative information estimated by the estimation unit 282 is not equal to or less than the predetermined value (No at Step S1013), the assistance device 200H proceeds to Step S1017 that will be described later.

At Step S1014, the acquisition unit 281 acquires the electrical information from the treatment tool control device 120 included in the treatment system 100.

Subsequently, the estimation unit 282 estimates the intraoperative information indicated during an operation on the basis of the first trained model, the electrical information that has been acquired by the acquisition unit 281, the quadratic discriminant information that has been estimated by the quadratic discriminant estimation unit 292, and the determination result obtained at Step S1013 described above (Step S1015).

The parameter control unit 284 changes the control parameter for controlling the treatment tool 110 performed by the treatment tool control device 120 to the control parameter that is included in the intraoperative information estimated by the estimation unit 282 at Step S1015 described above, and outputs the changed control parameter (Step S1016). For example, if the determination unit 283 determines, at Step S1013, that the pressure resistance value of the blood vessel is equal to or less than 500 mm Hg, and estimates, at Step S1015, that the size of the blood vessel that is included in the intraoperative information estimated by the estimation unit 282 is 4 mm, the parameter control unit 284 changes the control parameter to the control parameter corresponding to the pressure resistance value of the blood vessel equal to or less than 500 mm Hg and the size of the blood vessel of 4 mm, and outputs the changed control parameter to the treatment tool control device 120. After the process performed at Step S1016, the assistance device 200H proceeds to Step S1020 that will be described later.

At Step S1017, the acquisition unit 281 acquires the electrical information from the treatment tool control device 120 included in the treatment system 100.

Subsequently, the estimation unit 282 estimates the intraoperative information to be indicated during an operation on the basis of the first trained model, the electrical information that has been acquired by the acquisition unit 281, the quadratic discriminant information that has been estimated by the quadratic discriminant estimation unit 292, and the determination result obtained at Step S1013 described above (Step S1018).

The parameter control unit 284 changes the control parameter for controlling the treatment tool 110 performed by the treatment tool control device 120 to the control parameter that is included in the intraoperative information estimated by the estimation unit 282 at Step S1015 described above, and outputs the changed control parameter (Step S1019). For example, in a case where the determination unit 283 determines, at Step S1013, that the pressure resistance value of the blood vessel is not less than 500 mm Hg and estimates, at Step S1015, that the size of the blood vessel included in the intraoperative information estimated by the estimation unit 282 is 4 mm, if the pressure resistance value of the blood vessel is 1000 mm Hg, the parameter control unit 284 changes the control parameter to the control parameter corresponding to the pressure resistance value of the blood vessel of 1000 mm Hg and the size of the blood vessel of 4 mm, and then, outputs the changed control parameter to the treatment tool control device 120. After the process performed at Step S1016, the assistance device 200H proceeds to Step S1020 that will be described later.

At Step S1020, the determination unit 283 determines whether or not the operator has completed treatment performed on the subject. If it is determined, by the determination unit 283, that the operator has completed treatment performed on the subject (Yes at Step S1020), the assistance device 200H completes this process. In contrast, if it is determined, by the determination unit 283, that the operator has not completed treatment performed on the subject (No at Step S1020), the assistance device 200H returns to Step S1011 described above.

At Step S1021, the parameter control unit 284 maintains the control parameter for controlling the treatment tool 110 performed by the treatment tool control device 120. Specifically, the parameter control unit 284 maintains the control parameter that has been decided at Step S1002 described above without using the control parameter that is included in the intraoperative information estimated by the estimation unit 282. As a result of this, it is possible to prevent the control parameter from being automatically changed in a low reliable state of the estimation result when the accuracy of the estimation result estimated by the estimation unit 282 is less than the fixed value.

Subsequently, the determination unit 283 determines whether or not the operator has completed treatment performed on the subject. If it is determined, by the determination unit 283, that the operator has completed treatment performed on the subject (Yes at Step S1022), the assistance device 200H completes this process. In contrast, if it is determined, by the determination unit 283, that the operator has not completed treatment performed on the subject (No at Step S1022), the assistance device 200H returns to Step S1001 described above.

According to the above described first modification of the ninth embodiment, similarly to the above described ninth embodiment, the treatment tool control device 120 is able to supply, to the treatment tool 110, the high frequency energy signal and the ultrasound energy signal for performing optimum treatment on the target site that is being subjected to treatment by the treatment tool 110. As a result of this, the operator is able to perform optimum treatment on the target site by using the treatment tool 110.

### Second modification of the ninth embodiment

In the following, a second modification of the ninth embodiment will be described. The second modification of the ninth embodiment has the same configuration as that of the assistance device 200H according to the above described ninth embodiment, but the process to be performed is different. Accordingly, in the following description, a process performed by an assistance device according to the second modification of the ninth embodiment will be described. Furthermore, components that are the same as those included in the assistance device 200H according to the above described ninth embodiment is assigned the same reference numerals and descriptions thereof in detail will be omitted.

### Process performed by assistance device

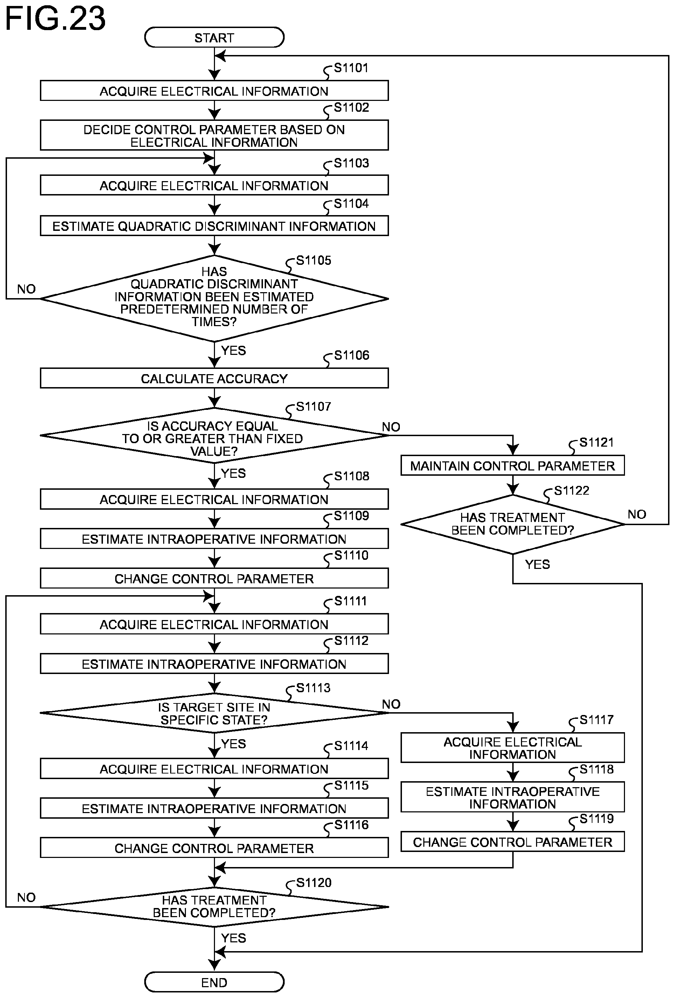

FIG. 23 is a flowchart illustrating an outline of the process performed by the assistance device 200H according to the second modification of the ninth embodiment. In FIG. 23, Step S1101 to Step S1112 and Step S1114 to Step S1122 correspond to Step S1001 to Step S1012 and Step S1014 to Step S1022, respectively, illustrated in FIG. 22 described above, and the same processes are performed at these steps; therefore, descriptions thereof in detail will be omitted. Accordingly, in the following description, each of the processes performed by the assistance device 200H at Step S1113 will be described.

At Step S1113, the determination unit 283 determines whether or not the state of the target site that is included in the intraoperative information estimated by the estimation unit 282 is in a specific state. For example, the determination unit 283 determines whether or not the state of the target site that is included in the intraoperative information estimated by the estimation unit 282 is in a bleeding state. In this case, the determination unit 283 determines that the state of the target site that is included in the intraoperative information estimated by the estimation unit 282 is in a specific state. If it is determined, by the determination unit 283, that the state of the target site that is included in the intraoperative information estimated by the estimation unit 282 is in the specific state (Yes at Step S1113), the assistance device 200H proceeds to Step S1114. In contrast, if it is determined, by the determination unit 283, that the state of the target site that is included in the intraoperative information estimated by the estimation unit 282 is not in the specific state (No at Step S1113), the assistance device 200H proceeds to Step S1117.

According to the above described second modification of the ninth embodiment, similarly to the above described ninth embodiment, the treatment tool control device 120 is able to supply, to the treatment tool 110, the high frequency energy signal and the ultrasound energy signal for performing optimum treatment on the target site that is being subjected to treatment by the treatment tool 110. As a result of this, the operator is able to perform optimum treatment on the target site by using the treatment tool 110.

### Tenth Embodiment

In the following, a tenth embodiment will be described. In the tenth embodiment, the function of an assistance device is provided in a treatment tool control device. Accordingly, in the following description, a configuration of a treatment tool control device according to the tenth embodiment will be described. Furthermore, components that are the same as those included in the assistance device 200 and 200A to 200H according to the above described first to the ninth embodiments is assigned the same reference numerals and descriptions thereof in detail will be omitted.

### Configuration of treatment tool control device

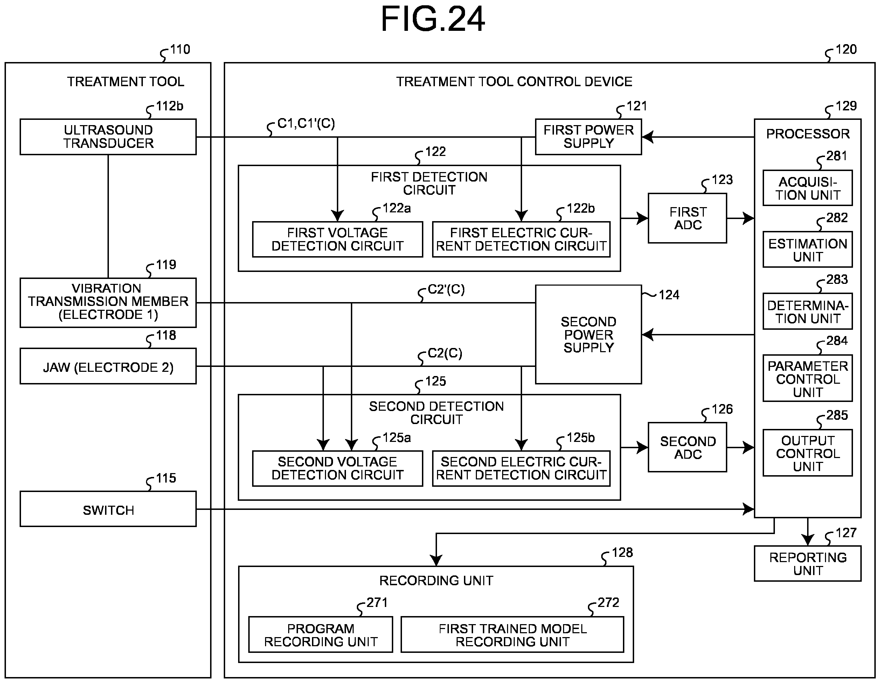

FIG. 24 is a block diagram illustrating the functional configuration of a treatment tool and a treatment tool control device. The treatment tool control device 120 illustrated in FIG. 24 includes a first power supply 121, a first detection circuit 122, a first Analog-to-Digital Converter (ADC) 123, a second power supply 124, a second detection circuit 125, a second ADC 126, a reporting unit 127, a recording unit 128, and a processor 129.

Here, a pair of transducer purpose lead wires C1 and C1' constituting the electrical cable 130 is connected to the ultrasound transducer 112b. Furthermore, in FIG. 24, for convenience of description, the pair of the transducer purpose lead wires C1 and C1' is illustrated by only using a single line. Further, the first power supply 121 outputs, to the ultrasound transducer 112b under the control of the processor 129, a driving signal that is electric power for generating ultrasound vibrations by way of the pair of the transducer purpose lead wires C1 and C1'. Accordingly, the ultrasound transducer 112b generates ultrasound vibrations. As a result of this, the vibration transmission member 119 functions as an electrode 1 by transmitting the ultrasound vibrations generated by the ultrasound transducer 112b from the end portion of the proximal end side Ar2 to the end portion 119a of the distal end side Ar1.

Furthermore, in the following description, for convenience of description, the driving signal that is output from the first power supply 121 to the ultrasound transducer 112b is referred to as an input driving signal, and the signal in which the input driving signal has been changed by a frequency response received from the ultrasound transducer 112b is referred to as an output driving signal.

The first detection circuit 122 includes a first voltage detection circuit 122a that is a voltage sensor for detecting a voltage value, and a first electric current detection circuit 122b that is an electric current sensor for detecting an electric current value, and temporally detects a US signal (analog signal) corresponding to the output driving signal. The US signal corresponds to an electrical characteristic value of the ultrasound transducer. Specifically, examples of the US signal include a phase signal of a voltage of an output driving signal (hereinafter, referred to as a "US voltage phase signal"), a phase signal of an electric current of the output driving signal (hereinafter, referred to as a "US electric current phase signal"), a phase difference between the voltage and the electric current of the output driving signal (hereinafter, referred to as a "US phase difference "), an electric current value of the output driving signal (hereinafter, referred to as a "US electric current"), a voltage value of the output driving signal (hereinafter, referred to as a "US voltage"), an electric power value of the output driving signal (hereinafter, referred to as "US electric power"), an impedance value calculated from the US electric current and the US voltage (hereinafter, referred to as a "ultrasound waves impedance value"), and the like.

The first ADC 123 converts the US signal (analog signal) that has been output from the first detection circuit 122 to a digital signal. Further, the first ADC 123 outputs the converted US signal (digital signal) to the processor 129.

Further, the second power supply 124 outputs, under the control of the processor 129, a high frequency electric current and a high frequency voltage between the jaw 118 and the vibration transmission member 119 via a pair of high frequency purpose lead wires C2 and C2'. That is, the jaw 118 functions as an electrode 2. Accordingly, a high frequency electric current flows into the target site that has been grasped between the jaw 118 and the end portion 119a (treatment portion) of the vibration transmission member 119. That is, high frequency energy is applied to the target site. Further, Joule heat is generated as a result of high frequency electric current flowing in the target site, so that the target site is subjected to treatment.

The second detection circuit 125 includes a second voltage detection circuit 125a that is a voltage sensor for detecting a voltage value, and a second electric current detection circuit 125b that is an electric current sensor for detecting an electric current value, and temporally detects a HF signal corresponding to a high frequency electric current and a high frequency voltage that are being output from the second power supply 124 to the jaw 118 and the end portion 119a. Specifically, examples of the HF signal includes a high frequency electric current (hereinafter, referred to as a "HF electric current") and a high frequency voltage (hereinafter, referred to as a "HF voltage") that are being output from the second power supply 124 to the jaw 118 and the end portion 119a, high frequency electric power that is calculated from the HF electric current and the HF voltage (hereinafter, referred to as "HF electric power"), an impedance value that is calculated from the HF electric current and the HF voltage (hereinafter, referred to as a "HF impedance value"), a phase difference between the HF electric current and the HF voltage (hereinafter, referred to as a "HF phase difference"), and the like.

The second ADC 126 converts the HF signal (analog signal) that has been output from the second detection circuit 125 to the digital signal. Further, the second ADC 126 outputs the converted HF signal (digital signal) to the processor 129.

The reporting unit 127 reports, under the control of the processor 129, predetermined information. Examples of the reporting unit 127 includes light emitting diode (LED) that reports predetermined information by lighting, flashing, or a color at the time of lighting, a display device that displays predetermined information, a speaker that outputs predetermined information by sound, and the like.

The recording unit 128 records various kinds of information related to the treatment tool control device 120. The recording unit 128 is configured by use of an HDD, an SSD, a flash memory, a volatile memory, a nonvolatile memory, or the like. The recording unit 128 includes the program recording unit 271 and the first trained model recording unit 272 that are the same as those included in the recording unit 270 according to the above described first embodiment.

The processor 129 is implemented by a processor including hardware, such as a GPU, a digital signal processor (DSP), an FPGA, or a CPU, and a memory that is a temporary storage area used by the processor. The processor 129 controls each of the units constituting the treatment tool control device 120. Furthermore, the processor 129 has the same function as that of the control unit 280 according to the above described first embodiment. Specifically, the processor 129 includes the estimation unit 282, the determination unit 283, the parameter control unit 284, and the output control unit 285. Furthermore, the processor 129 performs the same process as that performed in the above described first embodiment.

According to the above described tenth embodiment, if it is determined, by the determination unit 283, that the accuracy that is included in the intraoperative information estimated by the estimation unit 282 is equal to or greater than the fixed value, the parameter control unit 284 changes the control parameter for controlling the treatment tool 110 performed by the processor 129 on the basis of the intraoperative information that has been estimated by the estimation unit 282, and outputs the changed control parameter. As a result of this, the operator is able to appropriately control the treatment tool 110.

Furthermore, in the tenth embodiment, it may be possible to constitute the processor 129 by appropriately combining each of the functions of the control units 280A to 280H according to the above described second to the ninth embodiments, respectively, and perform the processes according to the second to the ninth embodiments.

### Other embodiments

Various embodiments may be made by appropriately combining a plurality of components disclosed in the medical system according to the first to the ninth embodiments in the above described present disclosure. For example, some components may be deleted from all of the components described in the medical system according to the first to the ninth embodiments in the above described present disclosure. Furthermore, the components described in the endoscope system according to the embodiments in the above described present disclosure may be appropriately combined.

Furthermore, in the medical system according to the first to the ninth embodiments in the present disclosure, the assistance device is connected to the endoscope system and the treatment system in a wired manner, but the embodiments are not limited to this. For example, the assistance device may be constituted in a server or the like, and may also be connected so as to be capable of performing bidirectional communication via a network.

Furthermore, in the assistance device according to the first to the ninth embodiments according to the present disclosure, the trained model is recorded in the recording unit, but the embodiment is not limited to this. For example, the trained model may be provided in a recording unit included in an external server, or the like.

Furthermore, in the medical system according to the first to the ninth embodiments described in the present disclosure, the "unit" described above may be replaced with a "means", a "circuit", or the like. For example, the control unit may be replaced with a control means or a control circuit.

Furthermore, the programs to be executed by the medical system according to the first to the ninth embodiments described in the present disclosure are provided by being recorded, as file data in an installable format or an executable format, on a computer readable recording medium such as a compact disk read only memory (CD-ROM), a flexible disk (FD), a compact disk recordable (CD-R), a digital versatile disk (DVD), a universal serial bus (USB) medium, or a flash memory.

Furthermore, the programs to be executed by the medical system according to the first to the ninth embodiments described in the present disclosure may be stored in a computer connected to a network, such as the Internet, and provided by being downloaded via the network.

Furthermore, in a description of the flowcharts in the application, the relationship between before and after the processes performed at each step is stated by using "first", "then", "subsequently", and the like; however, the order of the processes needed to implement the present invention is not uniquely determined by the descriptions above. Specifically, the order of the processes in the flowcharts described in the application may also be changed as long as processes do not conflict with each other.

In the above, embodiments of the present application have been described in detail based on the drawings; however the embodiments are described only by way of an example. In addition to the embodiments described in the present disclosure, the present disclosure can be implemented in a mode in which various modifications and changes are made in accordance with the knowledge of those skilled in the art.

According to the present disclosure, an advantage is provided in that it is possible to appropriately control the energy device.

Additional advantages and modifications will readily occur to those skilled in the art. Therefore, the disclosure in its broader aspects is not limited to the specific details and representative embodiments shown and described herein. Accordingly, various modifications may be made without departing from the spirit or scope of the general inventive concept as defined by the appended claims and their equivalents.

## Claims

1. An assistance device comprising:
a processor, the processor being configured to
acquire electrical information related to an energy signal for driving an energy device configured to apply energy to a site to perform treatment on the site, the site being a target for treatment in living tissue,
input the electrical information to a trained model to cause the trained model to estimate intraoperative information that is related to the site indicated during an operation, the trained model having been obtained by training a plurality of pieces of training data in each of which at least one of driving information obtained when the energy device is driven, device information indicating a state of the energy device, and site information related to a size, a type, and a state of the site is used as an input parameter and is associated with the intraoperative information that is used as an output parameter,
determine whether or not accuracy of the intraoperative information is equal to or greater than a fixed value, and
perform control according to the intraoperative information when it is determined that the accuracy is equal to or greater than the fixed value.

2. The assistance device according to claim 1, wherein the processor is configured to
calculate the accuracy of the intraoperative information,
determine whether or not the accuracy is equal to or greater than the fixed value, and
perform control according to the intraoperative information when it is determined that the accuracy is equal to or greater than the fixed value.

3. The assistance device according to claim 2, wherein the processor is configured to calculate the accuracy based on a plurality of pieces of intraoperative information.

4. The assistance device according to claim 1, wherein the processor is configured to output the intraoperative information externally when it is determined that the accuracy is equal to or greater than the fixed value.

5. The assistance device according to claim 1, wherein the processor is configured to
change a control parameter for driving the energy device based on the intraoperative information when it is determined that the accuracy is equal to or greater than the fixed value, and
maintain the control parameter for driving the energy device when it is determined that the accuracy is not equal to or greater than the fixed value.

6. The assistance device according to claim 1, wherein the electrical information is time series data related to at least one of a US signal and a HF signal for driving the energy device.

7. The assistance device according to claim 1, wherein the intraoperative information is at least one of the site information and a control parameter for controlling the energy device used when the energy device applies the energy to the site.

8. The assistance device according to claim 1, wherein the processor is configured to
input the electrical information to each of a plurality of trained models to cause each of the plurality of trained models to estimate a plurality of respective pieces of intraoperative information, each of the plurality of trained model having been obtained by training the plurality of respective pieces of training data that are obtained in different situations,
calculate the accuracy of each of the pieces of intraoperative information based on the plurality of respective pieces of intraoperative information,
determine whether or not the accuracy is equal to or greater than the fixed value, and
perform the control according to the intraoperative information when it is determined that the accuracy is equal to or greater than the fixed value.

9. The assistance device according to claim 1, wherein the processor is configured to
input the electrical information to an authentication trained model to cause the authentication trained model to estimate accuracy of the electrical information as an estimation result, the authentication trained model having been obtained by training a plurality of pieces of authentication training data in each of which at least one of the driving information obtained when the energy device is driven, the device information indicating the state of the energy device, and the site information related to the size, the type, and the state of the site is used as an input parameter and is associated with accuracy of deviation, which is used as an output parameter, between the electrical information and the plurality of pieces of training data,
determine whether or not the accuracy of the electrical information is equal to or greater than the fixed value, and
input the electrical information to the trained model to cause the trained model to estimate the intraoperative information when the accuracy of the electrical information is equal to or greater than the fixed value.

10. The assistance device according to claim 1, wherein the processor is configured to
input the electrical information to a determination trained model to cause the determination trained model to estimate association information indicating availability to the trained model, the determination trained model having been obtained by training a plurality of pieces of determination training data in each of which at least one of the driving information obtained when the energy device is driven, the device information indicating the state of the energy device, the site information related to the size, the type, and the state of the site, and the association information is used as an input parameter and is associated with the association information that is used as an output parameter,
determine whether or not the accuracy of the association information is equal to or greater than the fixed value, and
input the electrical information to the trained model to cause the trained model to estimate the intraoperative information when it is determined that the accuracy of the association information is equal to or greater than the fixed value.

11. The assistance device according to claim 1, wherein the processor is configured to
input the electrical information to a situation identification trained model to causes the situation identification trained model to estimate situation information that is related to a situation of the site, the situation identification trained model having been obtained by training a plurality of pieces of situation training data in each of which at least one of the driving information obtained when the energy device is driven, the device information indicating the state of the energy device, and the site information related to the size, the type, and the state of the site is used as an input parameter and is associated with the situation information that is used as an output parameter,
select an optimum trained model from among a plurality of trained models that have been obtained by training the plurality of pieces of training data obtained in different situations based on the situation information, and
input the electrical information to the optimum trained model to cause the optimum trained model to estimate the intraoperative information.

12. The assistance device according to claim 11, wherein the processor is configured to
determine whether or not accuracy of the situation information is equal to or greater than the fixed value, and
input the electrical information to the optimum trained model to cause the optimum trained model to estimate the intraoperative information when it is determined that the accuracy of the situation information is equal to or greater than the fixed value.

13. The assistance device according to claim 11, wherein the processor is configured to
input the electrical information to the optimum trained model to cause the optimum trained model to estimate the intraoperative information when it is determined that accuracy of the situation information is equal to or greater than the fixed value, and
maintain a control parameter for driving the energy device when it is determined that the accuracy of the situation information is not equal to or greater than the fixed value.

14. The assistance device according to claim 1, wherein the processor is configured to
input the electrical information to a grasp determination trained model to cause the grasp determination trained model to estimate grasping information related to a grasping state of the site grasped by the energy device, the grasp determination trained model having been obtained by training a plurality of pieces of grasp training data in each of which the grasping information and at least one of the driving information obtained when the energy device is driven, the device information indicating the state of the energy device, and the site information related to the size, the type, and the state of the site are used as input parameters and are associated with the grasping information that is used as an output parameter, and
further input the grasping information to the trained model to cause the trained model to estimate the intraoperative information.

15. The assistance device according to claim 14, wherein the processor is configured to
determine whether or not accuracy of the grasping information is equal to or greater than the fixed value, and
further input the grasping information to the trained model to cause the trained model to estimate the intraoperative information when it is determined that the accuracy of the grasping information is equal to or greater than the fixed value.

16. The assistance device according to claim 1, wherein the processor is considered to
decide, based on the electrical information, a control parameter for controlling the energy device, to output the decided control parameter,
acquire the electrical information after having output the control parameter to the energy device, and
input the electrical information to the trained model to cause the trained model to estimate the intraoperative information.

17. The assistance device according to claim 16, wherein the processor is configured to
input the electrical information to a quadratic discriminant trained model to cause the quadratic discriminant trained model to estimate quadratic discriminant information indicating whether or not quadratic discriminant is available by using the trained model, the quadratic discriminant trained model having been obtained by training a plurality of pieces of quadratic discriminant training data in each of which the quadratic discriminant information and at least one of the driving information obtained when the energy device is driven, the device information indicating the state of the energy device, and the site information related to the size, the type, and the state of the site are used as input parameters and are associated with the quadratic discriminant information that is used as an output parameter, and
input the electrical information and the quadratic discriminant information to the trained model to cause the trained model to estimate the intraoperative information.

18. The assistance device according to claim 17, wherein the quadratic discriminant information indicates either a pressure resistance value of a blood vessel at the site or a state of the treatment performed on the site by the energy device.

19. An assistance method implemented by an assistance device that comprises a processor, the processor being configured to
acquire electrical information related to an energy signal for driving an energy device configured to apply energy to a site to perform treatment on the site, the site being a target for treatment in living tissue,
input the electrical information to a trained model to cause the trained model to estimate intraoperative information that is related to the site indicated during an operation, the trained model having been obtained by training a plurality of pieces of training data in each of which at least one of driving information obtained when the energy device is driven, device information indicating a state of the energy device, and site information related to a size, a type, and a state of the site is used as an input parameter and is associated with the intraoperative information that is used as an output parameter,
determine whether or not accuracy of the intraoperative information is equal to or greater than a fixed value, and
perform control according to the intraoperative information when it is determined that the accuracy is equal to or greater than the fixed value.

20. A non-transitory computer-readable recording medium with an executable program stored thereon, the program causing a computer to execute:
acquiring electrical information related to an energy signal for driving an energy device configured to apply energy to a site to perform treatment on the site, the site being a target for treatment in living tissue;
inputting the electrical information to a trained model to cause the trained model to estimate intraoperative information that is related to the site indicated during an operation, the trained model having been obtained by training a plurality of pieces of training data in each of which at least one of driving information obtained when the energy device is driven, device information indicating a state of the energy device, and site information related to a size, a type, and a state of the site is used as an input parameter and is associated with the intraoperative information that is used as an output parameter;
determining whether or not accuracy of the intraoperative information is equal to or greater than a fixed value; and
performing control according to the intraoperative information when it is determined that the accuracy is equal to or greater than the fixed value.
